# EUROPEAN PATENT SPECIFICATION

(11) **EP 2 705 056 B1**
(45) Date of publication and mention of the grant of the patent: **14.11.2018**
(21) Application number: 12723732.9
(22) Date of filing: 08.05.2012
(51) Int. Cl.: C07K 16/22, C07K 16/46

(54) **ANTI-NERVE GROWTH FACTOR ANTIBODIES AND METHODS OF PREPARING AND USING THE SAME**
ANTI-NERVENWACHSTUMSFAKTOR-ANTIKÖRPER UND VERFAHREN ZU IHRER HERSTELLUNG UND VERWENDUNG
ANTICORPS CONTRE LE FACTEUR DE CROISSANCE NEURONALE ET LEURS PROCÉDÉS DE PRÉPARATION ET D'UTILISATION

(30) Priority: 06.05.2011 US 201161483491 P; 06.09.2011 US 201161531439 P
(43) Date of publication of application: 12.03.2014
(73) Proprietor: Nexvet Australia Pty Ltd, Melbourne VIC 3000 (AU)
(72) Inventor: GEARING, David, Southbank Victoria 3006 (AU)
(74) Representative: Wise, Daniel Joseph
(86) International application number: PCT/GB2012/051004
(87) International publication number: WO 2012/153123

(56) References cited:
- EP-A1- 0 417 486
- EP-A1- 2 138 512
- WO-A2-2006/131951
- WO-A2-2010/027488
- WO-A2-2012/024650
- PELAT THIBAUT ET AL: "Obtention and engineering of non-human primate (NHP) antibodies for therapeutics", MINI REVIEWS IN MEDICINAL CHEMISTRY, BENTHAM SCIENCE PUBLISHERS, HILVERSUM, NL, vol. 9, no. 14, 1 December 2009 (2009-12-01), pages 1633-1638, XP008134866, ISSN: 1389-5575, DOI: 10.2174/138955709791012283
- COVACEUSZACH S ET AL: "Dissecting NGF Interactions with TrkA and p75 Receptors by Structural and Functional Studies of an Anti-NGF Neutralizing Antibody", JOURNAL OF MOLECULAR BIOLOGY, ACADEMIC PRESS, UNITED KINGDOM, vol. 381, no. 4, 12 September 2008 (2008-09-12), pages 881-896, XP023611489, ISSN: 0022-2836, DOI: 10.1016/J.JMB.2008.06.008 [retrieved on 2008-06-10]
- Wolfgang F. Richter ET AL: "Animal Pharmacokinetics of the Tumor Necrosis Factor Receptor-Immunoglobulin Fusion Protein Lenercept and Their Extrapolation to Humans", Drug Metabolism and Disposition, 1 January 1999 (1999-01-01), page 21, XP055248479, UNITED STATES Retrieved from the Internet: URL:http://dmd.aspetjournals.org/content/2 7/1/21.long [retrieved on 2016-02-08]
- David Gareth Williams ET AL: "Humanising Antibodies by CDR Grafting", Antibody Engineering, 1 January 2010 (2010-01-01), pages 319-399, XP55369742, Retrieved from the Internet: URL:https://ebookcentral.proquest.com/lib/ epo-ebooks/home.action?ebraryDocId=null [retrieved on 2017-05-04]
- David Gareth Williams ET AL: "Humanising Antibodies by CDR Grafting", Antibody Engineering, 1 January 2010 (2010-01-01), pages 319-399, XP055369742, Retrieved from the Internet: URL:https://ebookcentral.proquest.com/lib/ epo-ebooks/home.action?ebraryDocId=null [retrieved on 2017-05-04]

### SEQUENCE LISTING

<110> NVIP Pty Ltd
<120> ANTI-NERVE GROWTH FACTOR ANTIBODIES AND METHODS OF PREPARING AND USING THE SAME
<130> P119713.WO.02
<150> US61/483491
   <151> 2011-05-06
<150> US61/531439
   <151> 2011-09-06
<160> 17
<170> PatentIn version 3.5
<210> 1
   <211> 107
   <212> PRT
   <213> Artificial Sequence
<220>
   <223> variable light chain (VL)
<400> 1
   Asp Ile Val Met Thr Gln Ser Pro Ala Ser Leu Ser Ala Ser Leu Gly
   1               5                   10                  15
   Glu Thr Val Thr Ile Glu Cys Arg Ala Ser Glu Asp Ile Tyr Asn Ala
               20                  25                  30
   Leu Ala Trp Tyr Gln Gln Lys Pro Gly Gln Ser Pro Lys Leu Leu Ile
           35                  40                  45
   Tyr Asn Thr Asp Thr Leu His Thr Gly Val Pro Ser Arg Phe Ser Gly
       50                  55                  60
   Ser Gly Ser Gly Thr Asp Tyr Ser Leu Thr Ile Asn Ser Leu Gln Ser
   65                  70                  75                  80
   Glu Asp Val Ala Ser Tyr Phe Cys Gln His Tyr Phe His Tyr Pro Arg
                   85                  90                  95
   Thr Phe Gly Gln Gly Thr Lys Leu Glu Leu Lys
               100                 105
<210> 2
   <211> 122
   <212> PRT
   <213> Artificial Sequence
<220>
   <223> variable heavy chain (VH)
<400> 2
   Gln Val Gln Leu Lys Glu Ser Gly Pro Gly Leu Val Asn Pro Ser Gln
   1               5                   10                  15
   Thr Leu Ser Leu Thr Cys Thr Val Ser Gly Phe Ser Leu Thr Asn Asn
               20                  25                  30
   Asn Val Asn Trp Val Arg Gln Ala Pro Gly Lys Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Ala Thr Ile Thr Arg Asp Thr Ser Lys Ser Gln Val Phe Leu
   65                  70                  75                  80
   Gln Met Asn Ser Leu Thr Ser Glu Asp Thr Ala Val Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Ile Leu Val Thr Val Ser Ser
           115                 120
<210> 3
   <211> 234
   <212> PRT
   <213> Artificial Sequence
<220>
   <223> full light chain - VL and Kappa light chain constant domain with leader sequence and 2 stop codons
<400> 3
   Met Gly Val Pro Thr Gln Leu Leu Gly Leu Leu Leu Leu Trp Ile Thr
   1               5                   10                  15
   Asp Ala Ile Cys Asp Ile Val Met Thr Gln Ser Pro Ala Ser Leu Ser
               20                  25                  30
   Ala Ser Leu Gly Glu Thr Val Thr Ile Glu Cys Arg Ala Ser Glu Asp
           35                  40                  45
   Ile Tyr Asn Ala Leu Ala Trp Tyr Gln Gln Lys Pro Gly Gln Ser Pro
       50                  55                  60
   Lys Leu Leu Ile Tyr Asn Thr Asp Thr Leu His Thr Gly Val Pro Ser
   65                  70                  75                  80
   Arg Phe Ser Gly Ser Gly Ser Gly Thr Asp Tyr Ser Leu Thr Ile Asn
                   85                  90                  95
   Ser Leu Gln Ser Glu Asp Val Ala Ser Tyr Phe Cys Gln His Tyr Phe
               100                 105                 110
   His Tyr Pro Arg Thr Phe Gly Gln Gly Thr Lys Leu Glu Leu Lys Arg
           115                 120                 125
   Asp Asp Ala Lys Pro Ser Ala Phe Ile Phe Pro Pro Ser Ser Glu Glu
       130                 135                 140
   Leu Ser Ser Gly Ser Ala Ser Val Val Cys Leu Val Tyr Gly Phe Tyr
   145                 150                 155                 160
   Pro Ser Gly Ala Thr Ile Asn Trp Lys Val Asp Gly Leu Ala Lys Thr
                   165                 170                 175
   Ser Ser Phe His Ser Ser Leu Thr Glu Gln Asp Ser Lys Asp Asn Thr
               180                 185                 190
   Tyr Ser Leu Ser Ser Thr Leu Thr Leu Pro Lys Ala Asp Tyr Glu Ala
           195                 200                 205
   His Asn Val Tyr Ala Cys Glu Val Ser His Lys Thr Leu Ser Ser Pro
       210                 215                 220
   Leu Val Lys Ser Phe Lys Arg Gln Asp Cys
   225                 230
<210> 4
   <211> 214
   <212> PRT
   <213> Artificial Sequence
<220>
   <223> light chain - variable and constant domains
<400> 4
   Asp Ile Val Met Thr Gln Ser Pro Ala Ser Leu Ser Ala Ser Leu Gly
   1               5                   10                  15
   Glu Thr Val Thr Ile Glu Cys Arg Ala Ser Glu Asp Ile Tyr Asn Ala
               20                  25                  30
   Leu Ala Trp Tyr Gln Gln Lys Pro Gly Gln Ser Pro Lys Leu Leu Ile
           35                  40                  45
   Tyr Asn Thr Asp Thr Leu His Thr Gly Val Pro Ser Arg Phe Ser Gly
       50                  55                  60
   Ser Gly Ser Gly Thr Asp Tyr Ser Leu Thr Ile Asn Ser Leu Gln Ser
   65                  70                  75                  80
   Glu Asp Val Ala Ser Tyr Phe Cys Gln His Tyr Phe His Tyr Pro Arg
                   85                  90                  95
   Thr Phe Gly Gln Gly Thr Lys Leu Glu Leu Lys Arg Asp Asp Ala Lys
               100                 105                 110
   Pro Ser Ala Phe Ile Phe Pro Pro Ser Ser Glu Glu Leu Ser Ser Gly
           115                 120                 125
   Ser Ala Ser Val Val Cys Leu Val Tyr Gly Phe Tyr Pro Ser Gly Ala
       130                 135                 140
   Thr Ile Asn Trp Lys Val Asp Gly Leu Ala Lys Thr Ser Ser Phe His
   145                 150                 155                 160
   Ser Ser Leu Thr Glu Gln Asp Ser Lys Asp Asn Thr Tyr Ser Leu Ser
                   165                 170                 175
   Ser Thr Leu Thr Leu Pro Lys Ala Asp Tyr Glu Ala His Asn Val Tyr
               180                 185                 190
   Ala Cys Glu Val Ser His Lys Thr Leu Ser Ser Pro Leu Val Lys Ser
           195                 200                 205
   Phe Lys Arg Gln Asp Cys
       210
<210> 5
   <211> 483
   <212> PRT
   <213> Artificial Sequence
<220>
   <223> full heavy chain - VH and HC2 (eqIgG2) with leader and stop codon
<400> 5
   Met Ala Val Leu Val Leu Leu Leu Cys Leu Val Thr Phe Pro Thr Cys
   1               5                   10                  15
   Val Leu Ser Gln Val Gln Leu Lys Glu Ser Gly Pro Gly Leu Val Asn
               20                  25                  30
   Pro Ser Gln Thr Leu Ser Leu Thr Cys Thr Val Ser Gly Phe Ser Leu
   35 40 45
   Thr Asn Asn Asn Val Asn Trp Val Arg Gln Ala Pro Gly Lys Gly Leu
   50 55 60
   Glu Trp Val Gly Gly Val Trp Ala Gly Gly Ala Thr Asp Tyr Asn Ser
   65 70 75 80
   Ala Leu Lys Ser Arg Ala Thr Ile Thr Arg Asp Thr Ser Lys Ser Gln
   85 90 95
   Val Phe Leu Gln Met Asn Ser Leu Thr Ser Glu Asp Thr Ala Val Tyr
   100 105 110
   Tyr Cys Ala Arg Asp Gly Gly Tyr Ser Ser Ser Thr Leu Tyr Ala Met
   115 120 125
   Asp Ala Trp Gly Gln Gly Ile Leu Val Thr Val Ser Ser Ala Ser Thr
   130 135 140
   Thr Ala Pro Lys Tyr Phe Gln Leu Thr Pro Ser Cys Gly Ile Thr Ser
   145 150 155 160
   Asp Ala Thr Val Ala Leu Gly Cys Leu Val Ser Asp Tyr Tyr Pro Glu
   165 170 175
   Pro Val Thr Val Ser Trp Asn Ser Gly Ala Leu Thr Ser Gly Val His
   180 185 190
   Thr Phe Pro Ser Val Leu Gln Ser Ser Gly Leu Tyr Ala Leu Ser Ser
   195 200 205
   Met Val Thr Val Pro Ala Ser Thr Trp Thr Ser Glu Thr Tyr Ile Cys
   210 215 220
   Asn Val Ala His Pro Ala Ser Ser Thr Lys Val Asp Lys Arg Ile Pro
   225 230 235 240
   Pro Cys Val Leu Ser Ala Glu Gly Val Ile Pro Ile Pro Ser Val Pro
   245 250 255
   Lys Pro Gln Cys Pro Pro Tyr Thr His Ser Lys Phe Leu Gly Gly Pro
   260 265 270
   Ser Val Phe Ile Phe Pro Pro Asn Pro Lys Asp Ala Leu Met Ile Ser
               275                 280                 285
   Arg Thr Pro Val Val Thr Cys Val Val Val Asn Leu Ser Asp Gln Tyr
       290                 295                 300
   Pro Asp Val Gln Phe Ser Trp Tyr Val Asp Asn Thr Glu Val His Ser
   305                 310                 315                 320
   Ala Ile Thr Lys Gln Arg Glu Ala Gln Phe Asn Ser Thr Tyr Arg Val
                   325                 330                 335
   Val Ser Val Leu Pro Ile Gln His Gln Asp Trp Leu Ser Gly Lys Glu
               340                 345                 350
   Phe Lys Cys Ser Val Thr Asn Val Gly Val Pro Gln Pro Ile Ser Arg
           355                 360                 365
   Ala Ile Ser Arg Gly Lys Gly Pro Ser Arg Val Pro Gln Val Tyr Val
       370                 375                 380
   Leu Pro Pro His Pro Asp Glu Leu Ala Lys Ser Lys Val Ser Val Thr
   385                 390                 395                 400
   Cys Leu Val Lys Asp Phe Tyr Pro Pro Asp Ile Ser Val Glu Trp Gln
                   405                 410                 415
   Ser Asn Arg Trp Pro Glu Leu Glu Gly Lys Tyr Ser Thr Thr Pro Ala
               420                 425                 430
   Gln Leu Asp Gly Asp Gly Ser Tyr Phe Leu Tyr Ser Lys Leu Ser Leu
           435                 440                 445
   Glu Thr Ser Arg Trp Gln Gln Val Glu Ser Phe Thr Cys Ala Val Met
       450                 455                 460
   His Glu Ala Leu His Asn His Phe Thr Lys Thr Asp Ile Ser Glu Ser
   465                 470                 475                 480
   Leu Gly Lys
<210> 6
   <211> 464
   <212> PRT
   <213> Artificial Sequence
<220>
   <223> full heavy chain - VH and HC2 (eqIgG2) w/o leader and stop codon
<400> 6
   Gln Val Gln Leu Lys Glu Ser Gly Pro Gly Leu Val Asn Pro Ser Gln
   1               5                   10                  15
   Thr Leu Ser Leu Thr Cys Thr Val Ser Gly Phe Ser Leu Thr Asn Asn
               20                  25                  30
   Asn Val Asn Trp Val Arg Gln Ala Pro Gly Lys Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Ala Thr Ile Thr Arg Asp Thr Ser Lys Ser Gln Val Phe Leu
   65                  70                  75                  80
   Gln Met Asn Ser Leu Thr Ser Glu Asp Thr Ala Val Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Ile Leu Val Thr Val Ser Ser Ala Ser Thr Thr Ala Pro
           115                 120                 125
   Lys Tyr Phe Gln Leu Thr Pro Ser Cys Gly Ile Thr Ser Asp Ala Thr
       130                 135                 140
   Val Ala Leu Gly Cys Leu Val Ser Asp Tyr Tyr Pro Glu Pro Val Thr
   145                 150                 155                 160
   Val Ser Trp Asn Ser Gly Ala Leu Thr Ser Gly Val His Thr Phe Pro
                   165                 170                 175
   Ser Val Leu Gln Ser Ser Gly Leu Tyr Ala Leu Ser Ser Met Val Thr
               180                 185                 190
   Val Pro Ala Ser Thr Trp Thr Ser Glu Thr Tyr Ile Cys Asn Val Ala
           195                 200                 205
   His Pro Ala Ser Ser Thr Lys Val Asp Lys Arg Ile Pro Pro Cys Val
       210                 215                 220
   Leu Ser Ala Glu Gly Val Ile Pro Ile Pro Ser Val Pro Lys Pro Gln
   225                 230                 235                 240
   Cys Pro Pro Tyr Thr His Ser Lys Phe Leu Gly Gly Pro Ser Val Phe
   245 250 255
   Ile Phe Pro Pro Asn Pro Lys Asp Ala Leu Met Ile Ser Arg Thr Pro
   260 265 270
   Val Val Thr Cys Val Val Val Asn Leu Ser Asp Gln Tyr Pro Asp Val
   275 280 285
   Gln Phe Ser Trp Tyr Val Asp Asn Thr Glu Val His Ser Ala Ile Thr
   290 295 300
   Lys Gln Arg Glu Ala Gln Phe Asn Ser Thr Tyr Arg Val Val Ser Val
   305 310 315 320
   Leu Pro Ile Gln His Gln Asp Trp Leu Ser Gly Lys Glu Phe Lys Cys
   325 330 335
   Ser Val Thr Asn Val Gly Val Pro Gln Pro Ile Ser Arg Ala Ile Ser
   340 345 350
   Arg Gly Lys Gly Pro Ser Arg Val Pro Gln Val Tyr Val Leu Pro Pro
   355 360 365
   His Pro Asp Glu Leu Ala Lys Ser Lys Val Ser Val Thr Cys Leu Val
   370 375 380
   Lys Asp Phe Tyr Pro Pro Asp Ile Ser Val Glu Trp Gln Ser Asn Arg
   385 390 395 400
   Trp Pro Glu Leu Glu Gly Lys Tyr Ser Thr Thr Pro Ala Gln Leu Asp
   405 410 415
   Gly Asp Gly Ser Tyr Phe Leu Tyr Ser Lys Leu Ser Leu Glu Thr Ser
   420 425 430
   Arg Trp Gln Gln Val Glu Ser Phe Thr Cys Ala Val Met His Glu Ala
   435 440 445
   Leu His Asn His Phe Thr Lys Thr Asp Ile Ser Glu Ser Leu Gly Lys
   450 455 460
<210> 7
   <211> 446
   <212> PRT
   <213> Artificial Sequence
<220>
   <223> - full heavy chain - VH and HC6 (eqIgG6) (without leader sequence and stop codons)
<400> 7
   Gln Val Gln Leu Lys Glu Ser Gly Pro Gly Leu Val Asn Pro Ser Gln
   1               5                   10                  15
   Thr Leu Ser Leu Thr Cys Thr Val Ser Gly Phe Ser Leu Thr Asn Asn
               20                  25                  30
   Asn Val Asn Trp Val Arg Gln Ala Pro Gly Lys Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Ala Thr Ile Thr Arg Asp Thr Ser Lys Ser Gln Val Phe Leu
   65                  70                  75                  80
   Gln Met Asn Ser Leu Thr Ser Glu Asp Thr Ala Val Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Ile Leu Val Thr Val Ser Ser Ala Ser Thr Thr Ala Pro
           115                 120                 125
   Lys Val Phe Gln Leu Ala Ser His Ser Ala Gly Thr Ser Asp Ser Thr
       130                 135                 140
   Val Ala Leu Gly Cys Leu Val Ser Ser Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ser Val Arg Gln Ser Ser Gly Leu Tyr Ser Leu Ser Ser Met Val Thr
               180                 185                 190
   Val Pro Ala Ser Ser Leu Lys Ser Gln Thr Tyr Ile Cys Asn Val Ala
           195                 200                 205
   His Pro Ala Ser Ser Thr Lys Val Asp Lys Arg Ile Val Ile Lys Glu
       210                 215                 220
   Pro Cys Cys Cys Pro Lys Cys Pro Gly Arg Pro Ser Val Phe Ile Phe
   225                 230                 235                 240
   Pro Pro Asn Pro Lys Asp Thr Leu Met Ile Ser Arg Thr Pro Glu Val
                   245                 250                 255
   Thr Cys Val Val Val Asp Val Ser Gln Glu Asn Pro Asp Val Lys Phe
               260                 265                 270
   Asn Trp Tyr Val Asp Gly Val Glu Ala His Thr Ala Thr Thr Lys Ala
           275                 280                 285
   Lys Glu Lys Gln Asp Asn Ser Thr Tyr Arg Val Val Ser Val Leu Pro
       290                 295                 300
   Ile Gln His Gln Asp Trp Arg Arg Gly Lys Glu Phe Lys Cys Lys Val
   305                 310                 315                 320
   Asn Asn Arg Ala Leu Pro Ala Pro Val Glu Arg Thr Ile Thr Lys Ala
                   325                 330                 335
   Lys Gly Glu Leu Gln Asp Pro Lys Val Tyr Ile Leu Ala Pro His Arg
               340                 345                 350
   Glu Glu Val Thr Lys Asn Thr Val Ser Val Thr Cys Leu Val Lys Asp
           355                 360                 365
   Phe Tyr Pro Pro Asp Ile Asn Val Glu Trp Gln Ser Asn Glu Glu Pro
       370                 375                 380
   Glu Pro Glu Val Lys Tyr Ser Thr Thr Pro Ala Gln Leu Asp Gly Asp
   385                 390                 395                 400
   Gly Ser Tyr Phe Leu Tyr Ser Lys Leu Thr Val Glu Thr Asp Arg Trp
                   405                 410                 415
   Glu Gln Gly Glu Ser Phe Thr Cys Val Val Met His Glu Ala Ile Arg
               420                 425                 430
   His Thr Tyr Arg Gln Lys Ser Ile Thr Asn Phe Pro Gly Lys
           435                 440                 445
<210> 8
   <211> 464
   <212> PRT
   <213> Artificial Sequence
<220>
   <223> VH and aglycosylated HC2 (without leader sequence and stop codons)
<400> 8
   Gln Val Gln Leu Lys Glu Ser Gly Pro Gly Leu Val Asn Pro Ser Gln
   1               5                   10                  15
   Thr Leu Ser Leu Thr Cys Thr Val Ser Gly Phe Ser Leu Thr Asn Asn
               20                  25                  30
   Asn Val Asn Trp Val Arg Gln Ala Pro Gly Lys Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Ala Thr Ile Thr Arg Asp Thr Ser Lys Ser Gln Val Phe Leu
   65                  70                  75                  80
   Gln Met Asn Ser Leu Thr Ser Glu Asp Thr Ala Val Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Ile Leu Val Thr Val Ser Ser Ala Ser Thr Thr Ala Pro
           115                 120                 125
   Lys Tyr Phe Gln Leu Thr Pro Ser Cys Gly Ile Thr Ser Asp Ala Thr
       130                 135                 140
   Val Ala Leu Gly Cys Leu Val Ser Asp Tyr Tyr Pro Glu Pro Val Thr
   145                 150                 155                 160
   Val Ser Trp Asn Ser Gly Ala Leu Thr Ser Gly Val His Thr Phe Pro
                   165                 170                 175
   Ser Val Leu Gln Ser Ser Gly Leu Tyr Ala Leu Ser Ser Met Val Thr
               180                 185                 190
   Val Pro Ala Ser Thr Trp Thr Ser Glu Thr Tyr Ile Cys Asn Val Ala
           195                 200                 205
   His Pro Ala Ser Ser Thr Lys Val Asp Lys Arg Ile Pro Pro Cys Val
       210                 215                 220
   Leu Ser Ala Glu Gly Val Ile Pro Ile Pro Ser Val Pro Lys Pro Gln
   225                 230                 235                 240
   Cys Pro Pro Tyr Thr His Ser Lys Phe Leu Gly Gly Pro Ser Val Phe
                   245                 250                 255
   Ile Phe Pro Pro Asn Pro Lys Asp Ala Leu Met Ile Ser Arg Thr Pro
   260 265 270
   Val Val Thr Cys Val Val Val Asn Leu Ser Asp Gln Tyr Pro Asp Val
   275 280 285
   Gln Phe Ser Trp Tyr Val Asp Asn Thr Glu Val His Ser Ala Ile Thr
   290 295 300
   Lys Gln Arg Glu Ala Gln Phe Ala Ser Thr Tyr Arg Val Val Ser Val
   305 310 315 320
   Leu Pro Ile Gln His Gln Asp Trp Leu Ser Gly Lys Glu Phe Lys Cys
   325 330 335
   Ser Val Thr Asn Val Gly Val Pro Gln Pro Ile Ser Arg Ala Ile Ser
   340 345 350
   Arg Gly Lys Gly Pro Ser Arg Val Pro Gln Val Tyr Val Leu Pro Pro
   355 360 365
   His Pro Asp Glu Leu Ala Lys Ser Lys Val Ser Val Thr Cys Leu Val
   370 375 380
   Lys Asp Phe Tyr Pro Pro Asp Ile Ser Val Glu Trp Gln Ser Asn Arg
   385 390 395 400
   Trp Pro Glu Leu Glu Gly Lys Tyr Ser Thr Thr Pro Ala Gln Leu Asp
   405 410 415
   Gly Asp Gly Ser Tyr Phe Leu Tyr Ser Lys Leu Ser Leu Glu Thr Ser
   420 425 430
   Arg Trp Gln Gln Val Glu Ser Phe Thr Cys Ala Val Met His Glu Ala
   435 440 445
   Leu His Asn His Phe Thr Lys Thr Asp Ile Ser Glu Ser Leu Gly Lys
   450 455 460
<210> 9
   <211> 446
   <212> PRT
   <213> Artificial Sequence
<220>
   <223> VH and aglycosylated HC6(without leader sequence and stop codons)
<400> 9
   Gln Val Gln Leu Lys Glu Ser Gly Pro Gly Leu Val Asn Pro Ser Gln
   1               5                   10                  15
   Thr Leu Ser Leu Thr Cys Thr Val Ser Gly Phe Ser Leu Thr Asn Asn
               20                  25                  30
   Asn Val Asn Trp Val Arg Gln Ala Pro Gly Lys Gly Leu Glu Trp Val
           35                  40                  45
   Gly Gly Val Trp Ala Gly Gly Ala Thr Asp Tyr Asn Ser Ala Leu Lys
       50                  55                  60
   Ser Arg Ala Thr Ile Thr Arg Asp Thr Ser Lys Ser Gln Val Phe Leu
   65                  70                  75                  80
   Gln Met Asn Ser Leu Thr Ser Glu Asp Thr Ala Val Tyr Tyr Cys Ala
                   85                  90                  95
   Arg Asp Gly Gly Tyr Ser Ser Ser Thr Leu Tyr Ala Met Asp Ala Trp
               100                 105                 110
   Gly Gln Gly Ile Leu Val Thr Val Ser Ser Ala Ser Thr Thr Ala Pro
           115                 120                 125
   Lys Val Phe Gln Leu Ala Ser His Ser Ala Gly Thr Ser Asp Ser Thr
       130                 135                 140
   Val Ala Leu Gly Cys Leu Val Ser Ser Tyr Phe Pro Glu Pro Val Thr
   145                 150                 155                 160
   Val Ser Trp Asn Ser Gly Ala Leu Thr Ser Gly Val His Thr Phe Pro
                   165                 170                 175
   Ser Val Arg Gln Ser Ser Gly Leu Tyr Ser Leu Ser Ser Met Val Thr
               180                 185                 190
   Val Pro Ala Ser Ser Leu Lys Ser Gln Thr Tyr Ile Cys Asn Val Ala
           195                 200                 205
   His Pro Ala Ser Ser Thr Lys Val Asp Lys Arg Ile Val Ile Lys Glu
       210                 215                 220
   Pro Cys Cys Cys Pro Lys Cys Pro Gly Arg Pro Ser Val Phe Ile Phe
   225                 230                 235                 240
   Pro Pro Asn Pro Lys Asp Thr Leu Met Ile Ser Arg Thr Pro Glu Val
                   245                 250                 255
   Thr Cys Val Val Val Asp Val Ser Gln Glu Asn Pro Asp Val Lys Phe
               260                 265                 270
   Asn Trp Tyr Val Asp Gly Val Glu Ala His Thr Ala Thr Thr Lys Ala
           275                 280                 285
   Lys Glu Lys Gln Asp Ala Ser Thr Tyr Arg Val Val Ser Val Leu Pro
       290                 295                 300
   Ile Gln His Gln Asp Trp Arg Arg Gly Lys Glu Phe Lys Cys Lys Val
   305                 310                 315                 320
   Asn Asn Arg Ala Leu Pro Ala Pro Val Glu Arg Thr Ile Thr Lys Ala
                   325                 330                 335
   Lys Gly Glu Leu Gln Asp Pro Lys Val Tyr Ile Leu Ala Pro His Arg
               340                 345                 350
   Glu Glu Val Thr Lys Asn Thr Val Ser Val Thr Cys Leu Val Lys Asp
           355                 360                 365
   Phe Tyr Pro Pro Asp Ile Asn Val Glu Trp Gln Ser Asn Glu Glu Pro
       370                 375                 380
   Glu Pro Glu Val Lys Tyr Ser Thr Thr Pro Ala Gln Leu Asp Gly Asp
   385                 390                 395                 400
   Gly Ser Tyr Phe Leu Tyr Ser Lys Leu Thr Val Glu Thr Asp Arg Trp
                   405                 410                 415
   Glu Gln Gly Glu Ser Phe Thr Cys Val Val Met His Glu Ala Ile Arg
               420                 425                 430
   His Thr Tyr Arg Gln Lys Ser Ile Thr Asn Phe Pro Gly Lys
           435                 440                 445
<210> 10
   <211> 23
   <212> PRT
   <213> Artificial Sequence
<220>
   <223> VL - FR1
<400> 10
   Asp Ile Val Met Thr Gln Ser Pro Ala Ser Leu Ser Ala Ser Leu Gly
   1               5                   10                  15
   Glu Thr Val Thr Ile Glu Cys
   20
<210> 11
   <211> 15
   <212> PRT
   <213> Artificial Sequence
<220>
   <223> VL - FR2
<400> 11
   Trp Tyr Gln Gln Lys Pro Gly Gln Ser Pro Lys Leu Leu Ile Tyr
   1 5 10 15
<210> 12
   <211> 32
   <212> PRT
   <213> Artificial Sequence
<220>
   <223> VL - FR3
<400> 12
   Gly Val Pro Ser Arg Phe Ser Gly Ser Gly Ser Gly Thr Asp Tyr Ser
   1 5 10 15
   Leu Thr Ile Asn Ser Leu Gln Ser Glu Asp Val Ala Ser Tyr Phe Cys
   20 25 30
<210> 13
   <211> 10
   <212> PRT
   <213> Artificial Sequence
<220>
   <223> VL - FR4
<400> 13
   Phe Gly Gln Gly Thr Lys Leu Glu Leu Lys
   1 5 10
<210> 14
   <211> 25
   <212> PRT
   <213> Artificial Sequence
<220>
   <223> VH - FR1
<400> 14
   Gln Val Gln Leu Lys Glu Ser Gly Pro Gly Leu Val Asn Pro Ser Gln
   1 5 10 15
   Thr Leu Ser Leu Thr Cys Thr Val Ser
               20                  25
<210> 15
   <211> 13
   <212> PRT
   <213> Artificial Sequence
<220>
   <223> VH - FR2
<400> 15
   Val Arg Gln Ala Pro Gly Lys Gly Leu Glu Trp Val Gly
   1               5                   10
<210> 16
   <211> 33
   <212> PRT
   <213> Artificial Sequence
<220>
   <223> VH - FR3
<400> 16
   Ser Arg Ala Thr Ile Thr Arg Asp Thr Ser Lys Ser Gln Val Phe Leu
   1               5                   10                  15
   Gln Met Asn Ser Leu Thr Ser Glu Asp Thr Ala Val Tyr Tyr Cys Ala
               20                  25                  30
   Arg
<210> 17
   <211> 11
   <212> PRT
   <213> Artificial Sequence
<220>
   <223> VH - FR4
<400> 17
   Trp Gly Gln Gly Ile Leu Val Thr Val Ser Ser
   1               5                   10

## Description

### Field of the Invention

The present disclosure relates to antibodies and fragments thereof which act as antagonists of equine nerve growth factor. The invention relates to methods of preparing same and to the therapeutic use of these antibodies and fragments in treating conditions associated with nerve growth factor such as pain, pain related disorders and conditions which result in the occurrence of pain in equines.

### Background to the Invention

Nerve growth factor (NGF) is a naturally occurring secreted protein which consists of an alpha, beta and gamma polypeptide chain. NGF is a member of the neurotrophin family and is implicated in a number of different roles. NGF promotes survival and differentiation of sensory and sympathetic neurons and signals via two membrane bound receptors, p75, a low affinity NGF receptor and TrkA, a transmembrane tyrosine kinase and a high affinity NGF receptor. The binding of NGF to TrkA or p75 results in an upregulation of neuropeptides in sensory neurons.

The use of NGF antagonists to treat pain and pain sensitivity in humans has been described (Cattaneo A., Curr. Op. Mol. Ther. 2010 12(1):94-106). For example, International Patent Application No. WO 2006/131951 describes a humanised form of the rat alphaD11 (αD11) monoclonal antibody. The αD11 antibody has binding specificity to mouse NGF, but is also known to bind to human and rat forms of NGF. Humanisation of the alphaD11 (αD11) rat derived monoclonal antibody is required prior to administration to humans in order to minimise the production of neutralising antibodies which result from a human anti-mouse antibody (HAMA) response being mounted against rodent derived antibodies. Furthermore, the replacement of mouse constant domains with human constant domains allows downstream effector functions to be selected for.

Pain management in equines is currently provided through administration of analgesic drugs of several classes, including local and general anaesthetics, opioid analgesics, α2 agonists, non-steroidal anti-inflammatory drugs (NSAIDs) and steroids. Each of these needs to be administered frequently and also has limitations in efficacy and safety. There is accordingly a need for an infrequently dosed, long lasting and efficacious form of pain relief for equines suffering from chronic pain, such as those with cancer pain or arthritis.

While NGF is known to be expressed in equine tissues, no antagonist to equine NGF has been described, nor has the use of blocking NGF mediated signalling in equines to prevent or alleviate pain. The use in equines of known antibodies which act as anti-NGF antagonists in other species would not be feasible due to the production of neutralising antibodies there against. Furthermore, the production of a chimeric antibody comprising equine derived constant domains and variable domains derived from a known anti-NGF antibody such as alphaD11 could not be guaranteed to bind to equine NGF. Furthermore, such an antibody may exhibit cross-reactivity to other target epitopes which may be present in equines, but not present in the species from which the antibody was originally derived. Furthermore, the production of neutralising antibodies there against would limit the long term administration of the antibody, this being a particularly important requirement when treating a chronic pain related condition or a cancerous condition. Likewise, the production of an equinised form of an anti-NGF antibody using CDR grafting, or a related technique may also result in neutralising antibody production and may further exhibit a reduction in antigen binding affinity and avidity. Accordingly, there is a serious need for binding members which act as antagonists of equine NGF and which retain high levels of binding affinity and avidity, while avoiding the production of neutralising antibodies there against, for use in pain management in equines.
WO2010/027488 describes monoclonal antibodies.
WO2006/131951 describes molecules that are able to inhibit the binding between NGF and the TrkA receptor as analgesics with prolonged effect.

Williams et al. (2010) Chapter 21 in Antibody Engineering Vol. 1 (Edited by Kontermann & Dübel) describe humanising antibodies by CDR grafting.

### Summary of the invention

Following extensive efforts, the present inventor has surprisingly produced equinised antibodies and binding fragments derived therefrom which bind specifically to equine NGF. It is demonstrated herein, quite unexpectedly, that the binding of the antibodies and binding fragments of the disclosure to equine NGF sequesters the biological activity of equine NGF by inhibiting the binding of equine NGF to the high affinity TrkA receptor or to the p75 receptor. This, in turn, prevents the upregulation of neuropeptides in sensory neurons with the resulting effect that the sensation of pain will be reduced or removed. The antibodies have been produced using recombinant DNA methods such that they are substantially non-immunogenic, that is, neutralising antibodies are not raised against them when administered to an equine subject. Such a finding is entirely surprising and unexpected, as the antibodies were not produced using standard methodologies, such as CDR grafting, or the like.

The invention provides a method of preparing an antibody suitable for use in an equine comprising
the steps of:
- providing a donor antibody from a species other than an equine, wherein the donor antibody has binding specificity for a target antigen present in equines;
- comparing each amino acid residue of the amino acid sequence of framework regions of the donor antibody with each amino acid residue present at a corresponding position in the amino acid sequence of framework regions of a plurality of equine antibodies to identify one or more amino acid residues within the amino acid sequence of the framework regions of the donor antibody that differ from one or more amino acid residues at the corresponding position within the amino acid sequence of framework regions of the plurality of equine antibodies; and
- substituting the one or more identified amino acid residues in the donor antibody with the one or more amino acid residues present at the corresponding position in the plurality of equine antibodies to produce a modified antibody,
wherein the modified antibody does not contain any amino acid in any position within the framework regions which would be foreign at the corresponding position in equines,
wherein the modified antibody is capable of specifically binding to equine nerve growth factor (NGF) and inhibiting the ability of equine NGF to bind to the p75 or TrkA equine NGF receptor; and
wherein substitution of an amino acid residue in the donor antibody is undertaken using the principle of conservative substitution.

The inventor has identified a process which modifies a donor antibody for use in an equine in such a way that the modified antibody does not contain any amino acid in any position within the framework regions which would be foreign at that position in equines. The modified antibody therefore retains the specificity and affinity of the donor antibody for the target antigen, but importantly is modified such that no potentially foreign epitopes are created. The modified antibody is therefore not seen as foreign in equines and hence does not induce an immune response in equines which could lead to a neutralisation of the efficacy of the antibody, especially following long term administration.

In certain embodiments, the step of substituting the one or more identified amino acid residues comprises substituting the one or more identified amino acid residues with the one or more amino acid residues present at the corresponding position which have the highest homology to the one or more substituted amino acid residues.

In certain embodiments, the method further comprises the step of replacing constant domains of the heavy chain and/or light chain of the donor antibody with constant domains of a heavy and/or light chain derived from an equine antibody.

Typically, the constant domain of the heavy chain is replaced with a type HC2 equine constant domain.
The target antigen is nerve growth factor (NGF).
The method of the invention does not comprise CDR grafting. Antibodies prepared according to the method of the invention comprise CDRs of the donor antibody, equinised framework regions prepared according to the method of the invention and equine constant domains.

Accordingly, according to a further aspect of the disclosure there is provided an equinised antibody or binding fragment thereof which binds specifically to equine neuronal growth factor (NGF). Typically, the equinised antibody or binding fragment thereof neutralises NGF biological function, when bound thereto. That is, the binding of the equinised antibody or binding fragment to NGF sequesters the ability of NGF to bind to the TrkA receptor or to the p75 receptor. In certain instances, the equinised antibody, or binding fragment thereof, binds to NGF with a binding affinity K_{D} of 1x10⁻⁸ or less. Typically, the equinised antibody is not immunogenic in equines.
The equinised antibody is prepared according to the method of preparing an antibody of the invention.
In a further or related aspect of the disclosure there is provided a neutralising antibody, or an antigen binding fragment thereof, which is capable of specifically binding to equine nerve growth factor (NGF), the antibody or antibody binding fragment comprising, consisting of or consisting essentially of a light chain variable region comprising the amino acid sequence of SEQ ID NO:1 or an amino acid sequence which has a sequence identity of at least 85, 90, 95 or 99% thereto. In certain instances said identity is over a length of at least about 15 amino acids, preferably about 20 amino acids, more preferably about 25 amino acids.

In some instances the neutralising antibody is a monoclonal antibody. In some instances, the antibody is a chimeric antibody. In some instances, the antibody is a equinised antibody, that is, an antibody which has an amino acid sequence which has been de-immunised such that neutralising antibodies will not be produced there against when administered to an equine subject. The equinised antibody is prepared according to the method of preparing an antibody of the invention. Typically the heavy chain constant domains of the antibody are selected or modified by way of amino acid substitution or deletion such that the constant domains do not mediate downstream effector functions. Typically said heavy chain is an equine HC2 or HC6 heavy chain. These isotypes have been shown to lack effector function (Lewis et al, Mol Immunol. 2008 Feb; 45(3): 818-827). Even more typically, said heavy chain is an equine HC2 heavy chain. This isotype has been shown by the present inventors to be purifiable by binding to Protein A.

In certain instances, the antibody or antibody binding fragment comprises, consists of, or consists essentially of a light chain comprising the amino acid sequence of SEQ ID NO:4 or an amino acid sequence which has at least 85, 90, 95 or 99% sequence homology thereto. In certain instances said identity is over a length of at least about 15 amino acids, preferably about 20 amino acids, more preferably about 25 amino acids.

In a further or related aspect, there is provided a neutralising antibody, or an antigen binding fragment thereof, which is capable of specifically binding to equine nerve growth factor (NGF), the antibody or antibody binding fragment comprising, consisting or consisting essentially of a heavy variable region comprising the amino acid sequence of SEQ ID NO:2 or an amino acid sequence which has a sequence identity of at least 85, 90, 95 or 99% thereto. In certain instances said identity is over a length of at least about 15 amino acids, preferably about 20 amino acids, more preferably about 25 amino acids.

Typically, the variable region of the heavy chain (VH) is conjoined to a further amino acid sequence which comprises at least one immunoglobulin constant domain. In certain instances, the immunoglobulin constant domain is derived from an antibody of the subclass IgG (immunoglobulin G) to form the complete heavy chain of the equinised antibody of the disclosure. Seven distinct equine immunoglobulin gamma (IgG) heavy chain constant domains are known. Typically, said constant domains comprise CH1, CH2 and CH3 along with a suitable linker (or "hinge") located between said CH1 and CH2 domains. Typically, the anti-equine NGF antibody of the disclosure comprising a heavy chain variable domain conjoined to a constant domain, wherein the constant domain does not mediate downstream effector functions such as complement fixation, ADCC, Fc receptor binding, or the like. Such heavy chains may comprise heavy chains having HC2 or HC6 isotypes and may have an amino acid sequence of SEQ ID NO:5, 6 or 7. Even more typically, said heavy chain is an equine HC2 heavy chain.

In certain instances, the antibody or antibody binding fragment comprises, consists of, or consists essentially of a heavy chain comprising the amino acid sequence of SEQ ID NO:6, which relates to the equine constant domain IgG2 (HC2) or to SEQ ID NO:7 which relates to equine constant domain IgG6 (HC6) or a sequence which has an amino acid identity of at least 85, 90, 95 or 99% thereto. In certain instances said identity is over a length of at least about 15 amino acids, preferably about 20 amino acids, more preferably about 25 amino acids.

In certain further instances, the antibody or binding fragment may comprise a heavy chain where at least one residue in the constant domain has been substituted or deleted in order to prevent the glycosylation of that residue. The deglycosylation of residues of the constant domain can limit downstream effector functions by preventing the binding of the constant domain (Fc domain) to Fc receptors (FcR) provided on cells. Accordingly, in certain further instances, the antibody or antibody binding fragment comprises, consists of, or consists essentially of a heavy chain comprising the amino acid sequence of SEQ ID NO:8 (aglycosylated version of IgG2 (HC2)) or SEQ ID NO:9 (aglycosylated version of IgG6 (HC6)) or an amino acid sequence which has a sequence identity of at least 95% thereto. In certain instances said identity is over a length of at least about 15 amino acids, preferably about 20 amino acids, more preferably about 25 amino acids.

In a further or related aspect, the present disclosure extends to a neutralising antibody, or an antigen binding fragment thereof, which is capable of specifically binding to equine nerve growth factor (NGF), the antibody or antibody binding fragment comprising, consisting or consisting essentially of a light chain and a heavy chain wherein the variable region of the light chain (VL) comprises an amino acid sequence of SEQ ID NO:1 or an amino acid sequence which has a sequence identity of at least 85, 90, 95 or 99% thereto and wherein the variable region of the heavy chain (VH) comprises, consists or consists essentially of an amino acid sequence which is identical or substantially homologous to the amino acid sequence of SEQ ID NO:2 or an amino acid sequence which has a sequence identity of at least 85, 90, 95 or 99% thereto. In certain instances said identity is over a length of at least about 15 amino acids, preferably about 20 amino acids, more preferably about 25 amino acids.

In certain instances, the antibody or binding member comprises a light chain which comprises, consists or consists essentially of the amino acid sequence of SEQ ID NO:4 or a sequence having an amino acid identity of at least 85%, more preferably 95% and more preferably at least 98% identity thereto. In certain instances said identity is over a length of at least about 15 amino acids, preferably about 20 amino acids, more preferably about 25 amino acids.

In certain instances, the antibody or binding member comprises a heavy chain which comprises, consists of or consists essentially of an amino acid sequence of SEQ ID NO:5, SEQ ID NO:6 or SEQ ID NO:7 or a sequence having an identity of at least 85%, more preferably 90% and most preferably at least 98% identity thereto. In certain instances said identity is over a length of at least about 15 amino acids, preferably about 20 amino acids, more preferably about 25 amino acids.

Typically the heavy chain constant domains of the antibody are selected or modified by way of amino acid substitution or deletion such that the constant domains do not mediate downstream effector functions. Typically said heavy chain is an equine HC2 or HC6 heavy chain. Even more typically, said heavy chain is an equine HC2 heavy chain. In certain instances, the antibody or binding member comprises a heavy chain which comprises, consists of or consists essentially of an amino acid sequence of SEQ ID NO:5 or SEQ ID NO:6, or a sequence having an identity of at least 85%, more preferably 90% and most preferably at least 98% identity thereto. In certain instances said identity is over a length of at least about 15 amino acids, preferably about 20 amino acids, more preferably about 25 amino acids. SEQ ID NO:5 and SEQ ID NO:6 comprise HC2 heavy chains, which have been shown to lack effector function, but can be purified using Protein A columns. This allows antibodies having HC2 heavy chains to be purified at a large scale in manufacturing and is thus advantageous.

In certain embodiments, the antibody may be conjugated to at least one reporter molecule.

In certain further embodiments at least one residue in the constant domain can be substituted or deleted in order to prevent the glycosylation of that residue. Accordingly, in certain further instances, the antibody or antibody binding fragment comprises, consists of, or consists essentially of a heavy chain comprising the amino acid sequence of SEQ ID NO:8 or SEQ ID NO:9 or a sequence having an identity of at least 85%, more preferably 90% and most preferably at least 98% identity thereto. In certain instances said identity is over a length of at least about 15 amino acids, preferably about 20 amino acids, more preferably about 25 amino acids.

The inventor has further defined a series of framework regions (FR) which can be combined with complementarity determining regions (CDRs) to form equinised heavy and light chain variable domains. Each of the equine heavy and light chain domains has 4 framework regions, designated FR1, FR2, FR3 and FR4.

An antibody molecule may comprise a heavy chain variable domain comprising CDR1, CDR2 and CDR3 regions and associated interposed framework regions. The heavy chain variable domain (VH) CDRs are known as HCDRs, with these CDRs being found at the following positions according to the Kabat numbering system: HCDR1 - Kabat residues 31-35, HCDR2 - Kabat residues 50-65, HCDR3 - Kabat residues 95-102 (Kabat EA et al. (1991) Sequences of proteins of immunological interest, 5th edition. Bethesda: US Department of Health and Human Services).

Furthermore, an antibody further comprises a light chain variable domain comprising CDR1, CDR2 and CDR3 regions and associated interposed framework regions. The light chain variable domain (VL) CDRs are known as LCDRs, with these CDRs being found at the following positions according to the Kabat numbering system: LCDR1 - Kabat residues 24-34, LCDR2 - Kabat residues 50-56, LCDR3 - Kabat residues 89-97.

A light or heavy chain variable domain comprises four framework regions, FR1, FR2, FR3 and FR4, interposed with CDRs in the following arrangement: FR1-CDR1-FR2-CDR2-FR3-CDR3-FR4.

In a further or related aspect, the present disclosure extends to an anti-NGF antibody, or an NGF antigen binding fragment thereof, the antibody or antibody binding fragment comprising a light chain variable region comprising at least one of:
an FR1 framework region consisting or comprising of the amino acid sequence of SEQ ID NO:10
an FR2 framework region consisting or comprising of the amino acid sequence of SEQ ID NO:11,
an FR3 framework region consisting or comprising of the amino acid sequence of SEQ ID NO:12, and
an FR4 framework region consisting or comprising of the amino acid sequence of SEQ ID NO:13
and/or a heavy chain variable region comprising at least one of:
an FR1 framework region consisting or comprising of the amino acid sequence of SEQ ID NO:14,
an FR2 framework region consisting or comprising of the amino acid sequence of SEQ ID NO:15,
an FR3 framework region consisting or comprising of the amino acid sequence of SEQ ID NO:16, and
an FR4 framework region consisting or comprising of the amino acid sequence of SEQ ID NO:17.

Typically the light and heavy chain CDRs are derived from an antibody which has binding specificity to NGF, preferably equine NGF.

Typically, the production of the equinised anti-equine NGF antibody of the disclosure does not require back mutations to be introduced into the framework regions of the light or heavy chain variable domains.

In certain instances, the light chain variable domain comprising said at least one framework region described above is conjoined to an equine derived light chain constant domain, typically a light chain kappa constant domain, but optionally a lambda light chain. In certain instances, said light chain comprises an FR1 region with an amino acid sequence of SEQ ID NO:10, an FR2 region with an amino acid sequence of SEQ ID NO:11, an FR3 region with an amino acid sequence of SEQ ID NO:12, and an FR4 region with an amino acid sequence of SEQ ID NO:13 or a framework region with an amino acid sequence which has a sequence identity of at least 85, 90, 95 or 98% to the foregoing. In certain instances said identity is over a length of at least about 5 amino acids, preferably about 10 amino acids.

In certain further instances, the heavy chain variable region comprising at least one of the framework regions described above is conjoined to an equine derived heavy chain constant domain. In certain instances, the amino acid sequence of the constant domain lacks any post-translational modifications, or may be modified to remove any or all residues which may be subject to N-linked glycosylation or O-linked glycosylation, such that the constant domains are aglycosylated. In certain instances the heavy chain comprises an FR1 region with an amino acid sequence of SEQ ID NO:14, an FR2 region with an amino acid sequence of SEQ ID NO:15, an FR3 region with an amino acid sequence of SEQ ID NO:16 and an FR4 region with an amino acid sequence of SEQ ID NO:17 or a framework region with an amino acid sequence which has a sequence identity of at least 85, 90, 95 or 98% to the foregoing. In certain instances said identity is over a length of at least about 5 amino acids, preferably about 10 amino acids.

In certain further embodiments, modifications may be made to the framework regions described herein. That is, the inventor has identified that for some residues in each framework region, there is a choice of amino acids for a given position. Importantly, these framework region modifications do not result in a conformational change to the complementarity determining regions, as this may alter the binding specificity and/or affinity of the resulting antibody. In certain embodiments, the invention extends to introducing 2 or more amino acid substitutions to the amino acid residues of the framework regions of the light chain variable region and/or heavy chain variable region.

Accordingly, in certain further instances, the disclosure extends to polypeptides, such as an antibody, or antigen binding fragment thereof, which comprises a light chain variable domain having an FR1 region comprising the amino acid sequence of SEQ ID NO:10 which has been modified by one or more of the following amino acid substitutions (where the amino acids are denoted by their single letter code): amino acid residue I at position 2 (12) is replaced by the amino acid residue V, S7 is T, A9 is E, L11 is V, S12 is T or A, A13 is V, S14 is T, E17 is Q, T18 is R, T20 is E, 121 is I, L, M or V and E22 is K. Furthermore, one or more of the following substitutions may further be provided: D1 is G, K or V, I2 is F, N, S or T, V3 is A, G, I or M, M4 is L, Q or V, T5 is A or I, S7 is F, A9 is D, P or S, S10 is F, L or T, L11 is S, S12 is E or V, A13 is L, Q or T, S14 is A or P, L15 is P or R, G16 is R, T18 is S, G or K, V19 is A, T20 is D or V, 121 is T and E22 is L, N, Q, R, S or T.

In certain further instances, the light chain FR2 region having the amino acid sequence of SEQ ID NO:11 may be modified by one or more of the following amino acid substitutions: K5 is R, Q8 is E, S9 is A, K11 is R or E, L12 is R. Furthermore, one or more of the following substitutions may further be provided: Y2 is F or H, Q3 is R or S, Q4 is H, K, R or V, K5 is V, P6 is I, L or S, S9 is P, R, V or T, P10 is L, K11 is I or L, L12 is A, E, G, H, Q, or W, L13 is F, I, M or V, 114 is F, T, M or V and Y15 is A, C, D, E, F, G, H, Q, R, S, T or V.

In certain further instances, the light chain FR3 region having the amino acid sequence of SEQ ID NO:12 may be modified by one or more of the following amino acid substitutions: S4 is D, F6 is Y, D14 is E, Y15 is F, S16 is T, N20 is S, S24 is A, S29 is I, S or T and F31 is Y. Furthermore, one or more of the following substitutions may further be provided: G1 is D or F, V2 is A or F, P3 is L or S, S4 is A, E, G or L, F6 is L, S7 is C, F, G, N, R or T, G8 is A, S9 is D, E, G, K, R, T or W, G10 is A, R or V, S11 is A, F, T or Y, G12 is E or T, T13 is A, S or W, S16 is A or V, L17 is F or P, T18 is A, I, S or V, 119 is V, N20 is D, G or T, S21 is D, E, P, R or T, Q23 is E or R, S24 is E or T, E25 is A, D, G or T, D26 is N, V27 is A, L, E, G or S, A28 is G, S29 is D, E, F, L, M, N or V, Y30 is C and F31 is H, S, T, V or W.

In certain further instances, the light chain FR4 region having the amino acid sequence of SEQ ID NO:13 may be modified by the following amino acid substitution: L9 is I. Furthermore, one or more of the following substitutions may further be provided: F1 is I or L, Q3 is L, T5 is S, K6 is M, N or R, L7 is M or V, E8 is A, D or K, L9 is F, M or V and K10 is A, E, G, I, Q, R, T or V.

In certain further instances, the heavy chain FR1 region having the amino acid sequence of SEQ ID NO:14 may be modified by the following amino acid substitution: N13 can be K. Furthermore, one or more of the following substitutions may further be provided: K5 can be Q, G10 can be D, L11 can be Q, V12 can be M, N13 can be M or R, P14 can be I or S, S15 can be A or G, Q16 can be E, T17 can be A, S19 can be T, T21 can be S or V, T23 can be A, F or S, V24 can be I, S25 can be T, G26 can be AF27 can be A, G, I, M, N Q or S, S28 can be D, H, I, L, N or P, L29 can be D, S, T or V and T30 can be E, I, N or R.

In certain further instances, the heavy chain FR2 region having the amino acid sequence of SEQ ID NO:15 may be modified by the following amino acid substitution: W12 is F. Furthermore, one or more of the following substitutions may further be provided: V2 can be L, A5 can be P, S or V, K8 can be W, G9 can be R, L10 can be P or W, W12 can be E, H, R, V or Y and G14 can be A, D or S.

In certain further instances, the heavy chain FR3 region having the amino acid sequence of SEQ ID NO:16 may be modified by one or more of the following amino acid substitutions: T3 is S, R6 is K, F14 is Y, Q16 is T, M17 is L, R32 is G. Furthermore, A2 can be C, G, I, T or V, T3 can be D, I, M N or R, I4 is V, T5 is I, L or S, R6 is E or S, D7 is E or N, T8 is A, E, I, P, S or Y, S9 is E, G, K or T, K10 is E, L, N, Q or R, S11 is G, K, N or R, Q12 is E, H or R, V13 is A, I, L, F or S, F14 is L, R,S, T or V, L15 is V, Q16 is I, M17 is V, N18 is D, K, R, SorT, S19 is D, E, G, K, M or T, L20 is M or V, T21 is S, S22 is D, E, G or R, E23 is D or G, T25 is A, A26 is S, V27 is D, Y29 is A, F, I or W, A31 is E, G, I, S, T or V and R32 is A, E, G, H, I, K or S.

In certain further instances, the heavy chain FR4 region having the amino acid sequence of SEQ ID NO:17 may be modified by the following amino acid substitution: Q3 is P.

In certain instances of the above aspects of the disclosure, the antibody is a monoclonal antibody. Typically the antibody is an equinised antibody.

In certain further instances of the above aspects of the disclosure, the equinised NGF neutralising antibody of the disclosure, or the binding fragment derived therefrom specifically binds to equine NGF (nerve growth factor) with a binding affinity having an equilibrium dissociation constant (K_{D}) of 1x10⁻⁸ or less. Furthermore, it is preferred that the equinised antibodies are not cross-reactive to any other epitopes present in equines, and further that neutralising antibodies are not generated against the antibodies of the disclosure when they are administered to an equine. Furthermore, it is preferred that the constant domains of the antibodies do not mediate any downstream effector functions including, but not limited to, complement fixation and activation, ADCC and Fc receptor binding and activation.

In certain further embodiments, modifications to the amino acid sequence of the constant regions of the heavy chain may be made to the antibodies of the invention. Said modification may involve the addition, substitution or deletion of one or more amino acid residues. Said amino acid changes are typically performed in order to modify the functional characteristics of the antibody. For example, amino acid modification may be performed to prevent downstream effector functions mediated by the antibody constant domains, for example by preventing the ability of the antibody to bind to Fc receptors, activate complement or induce ADCC. Furthermore, modifications may be made to the hinge region of the heavy chain constant domain in order to modify the circulatory half life of an antibody when it is administered to an equine.

Typically the heavy chain constant domains of the antibody are selected or modified by way of amino acid substitution or deletion such that the constant domains do not mediate downstream effector functions. Typically said heavy chain is an equine HC2 or HC6 heavy chain. Even more typically, said heavy chain is an equine HC2 heavy chain.

In a further or related aspect, the disclosure extends to an antibody or binding fragment thereof which specifically binds to one or more equine soluble proteins wherein the antibody does not mediate downstream effector functions and wherein the antibody is purifiable by binding to Protein A.

In certain instances, the one or more soluble proteins is selected from the group consisting of CSF, interleukins, growth factors and neurotrophins. In certain instances, the one or more soluble proteins is a neurotrophin. In certain instances, the one or more soluble proteins is NGF.

In certain instances, the antibody comprises a heavy chain which has been selected or modified by way of amino acid substitution or deletion such that the antibody does not mediate downstream effector functions.

In certain instances, the antibody comprises a heavy chain having a HC2 isotype. Antibodies comprising HC2 isotypes have been shown to lack effector function and to be purifiable using a Protein A column or Protein A affinity chromatography.

In certain instances, the antibody is an antibody which has been obtained following purification by binding to Protein A, e.g. using a Protein A column or Protein A affinity chromatography.

In certain instances, the antibody comprises a light chain and a heavy chain wherein the variable region of the light chain (VL) comprises an amino acid sequence of SEQ ID NO:1 or an amino acid sequence which has a sequence identity of at least 85, 90, 95 or 99% thereto and wherein the variable region of the heavy chain (VH) comprises, consists or consists essentially of an amino acid sequence which is identical or substantially homologous to the amino acid sequence of SEQ ID NO:2 or an amino acid sequence which has a sequence identity of at least 85, 90, 95 or 99% thereto. In certain instances said identity is over a length of at least about 15 amino acids, preferably about 20 amino acids, more preferably about 25 amino acids.

In certain instances, the antibody comprises a light chain which comprises, consists or consists essentially of the amino acid sequence of SEQ ID NO:4 or a sequence having an amino acid identity of at least 85%, more preferably 95% and more preferably at least 98% identity thereto. In certain instances said identity is over a length of at least about 15 amino acids, preferably about 20 amino acids, more preferably about 25 amino acids.

In certain instances, the antibody comprises a heavy chain which comprises, consists of or consists essentially of an amino acid sequence of SEQ ID NO:5 or SEQ ID NO:6 or a sequence having an identity of at least 85%, more preferably 90% and most preferably at least 98% identity thereto. In certain instances said identity is over a length of at least about 15 amino acids, preferably about 20 amino acids, more preferably about 25 amino acids.

In certain instances, the antibody is a monoclonal antibody. Typically the antibody is an equinised antibody.

In certain further instances, the antibody of the disclosure, or the binding fragment derived therefrom specifically binds to equine NGF (nerve growth factor) with a binding affinity having an equilibrium dissociation constant (K_{D}) of 1x10⁻⁸ or less. Furthermore, it is preferred that the antibodies are not cross-reactive to any other epitopes present in equines, and further that neutralising antibodies are not generated against the antibodies of the disclosure when they are administered to an equine.

In certain instances, the antibody, or antigen binding fragment thereof, does not mediate downstream effector functions. Typically the antibody or binding fragment has an equine heavy chain subtype HC2.

The equinised antibody is prepared according to the method of preparing an antibody of the invention.

The present disclosure extends to antibody fragments which bind to equine NGF and sequester its ability to bind to the equine p75 and TrkA receptors.

In certain instances the antibody binding fragment of any of the antibodies of the disclosure may comprise a heavy chain and light chain sequence of the disclosure being connected by a flexible linker to form a single chain antibody.

A single chain Fv (scFv) comprises a VH and VL domain. The VH and VL domains associate to form a target binding site. These 2 domains are covalently linked by a peptide linker. A scFv molecule can have the form of VL-linker-VH, in cases where the light chain variable domain is required at the N-terminal, or as VH-linker-VL in cases where the VH domain is required at the N-terminal. Accordingly, in certain further instances, the antigen binding fragment is a single chain Fv (scFv) antibody fragment. In certain further instances, the antibody binding fragment is selected from the group consisting of, but not limited to, a Fab antibody fragment, a Fab' antibody fragment, an F(ab')₂ antibody fragment, an Fv antibody fragment, a sFV antibody fragment, and the like.

In certain further instances, the disclosure provides multispecific or multivalent antibodies comprising an anti-NGF antibody or binding fragment of the disclosure coupled or conjoined to other antibodies with different binding specificities for use in combination therapy. A multispecific antibody comprises at least one antibody or binding fragment specific to a first NGF epitope, and at least one binding site specific to another epitope present on equine NGF, or to a different antigen. A multivalent antibody comprises antibodies or antibody binding fragments which have binding specificity to the same equine NGF epitope. Accordingly, in certain instances, the disclosure extends to an antibody fusion protein comprising four or more Fv regions or Fab regions of the equinised antibodies of the present disclosure. A yet further instance extends to an antibody fusion protein comprising one or more Fab region derived from an antibody described herein along with one or more Fab or Fv regions from antibodies specific for equine NGF. In certain further instances, the disclosure extends to a bispecific antibody, wherein an antibody or binding fragment thereof according to the present disclosure is linked to a secondary antibody or binding fragment thereof which has binding specific for a secondary target, said target not being equine NGF. Preferably said secondary target assists in preventing NGF mediated signalling through the p75 or TrkA receptors. Such multivalent, bispecific or multispecific antibodies can be made by a variety or recombinant methods which would be well known to the person skilled in the art.

In a yet further aspect of the disclosure there is provided an anti-neurotrophin neutralising antibody comprising a light chain variable domain having the amino acid sequence of SEQ ID NO:1 and/or a heavy chain variable domain having the amino acid sequence of SEQ ID NO:2. In certain instances, the neurotrophin is equine nerve growth factor (NGF).

A yet further aspect of the disclosure provides a method for treating, inhibiting or ameliorating pain in an equine, the method comprising the steps of:
- providing a therapeutically effective amount of an anti-equine NGF antibody, or antigen binding fragment thereof, wherein the antibody is an equinised antibody,
- administering the same to an equine in need thereof.

In certain instances, the equinised antibody comprises a light chain variable domain comprising the amino acid sequence of SEQ ID NO:1 or a sequence which has at least 95% identity thereto and/or a heavy chain variable domain comprising the amino acid sequence of SEQ ID NO:2 or an amino acid sequence having at least 95% sequence homology thereto.

In certain instances, the equinised antibody comprises a light chain having the amino acid sequence of SEQ ID NO:4 or a sequence having a sequence identity of at least 95% thereto and/or a heavy chain which comprises, consists of or consists essentially of an amino acid sequence selected from the group consisting of SEQ ID NO:5, SEQ ID NO:6, SEQ ID NO:7, SEQ ID NO:8 or SEQ ID NO:9 and a sequence having an amino acid identity of at least 95% and more preferably at least 98% identity to the foregoing.

In certain instances, the equinised antibody or antigen binding fragment thereof is any of those provided by the foregoing aspects of the disclosure.

In certain instances, the pain is neuropathic pain. In particular, the pain may be post-operative or post-surgical pain. Post-operative pain may result following any operating procedure which in equines may include, but is not limited to, orthopaedic surgery, soft tissue surgery, ovariohysterectomy procedures and the like. In certain further instances, the pain is chronic pain associated with cancer or a cancerous condition (oncologic pain). In certain further instances, the pain is associated with, or resulting from, inflammation, pruritis, rheumatoid arthritis or osteoarthritis. In certain further instances, the pain is associated with, or resulting from, palmar foot pain, subsolar bruising, laminitis, hoof abscesses, post showing trauma, post race trauma, navicular syndrome and proximal suspensory desmitis.

According to a yet further aspect of the present disclosure there is provided a method for the treatment of arthritis in an equine subject, said method comprising the steps of:
- providing a therapeutically effective amount of an anti-equine NGF antibody according to the disclosure or an antigen binding fragment thereof, and
- administering the same to an equine in need thereof.

In certain instances, the antibody is an equinised antibody. In certain instances, the equinised antibody comprises a light chain variable domain comprising the amino acid sequence of SEQ ID NO:1 or SEQ ID NO:3 or a sequence which has at least 85% identity thereto and/or a heavy chain variable domain comprising the amino acid sequence of SEQ ID NO:2 or SEQ ID NO:4 or an amino acid sequence having at least 85% sequence homology thereto.

In certain instances, arthritis or arthritic condition includes the conditions selected from the group consisting of immune mediated polyarthritis, rheumatoid arthritis, osteoarthritis and related conditions.

Typically, the treatment of the arthritis or arthritic condition comprises ameliorating, inhibiting, reducing, suppressing or delaying the onset of pain associated with, or attributable to, the arthritic condition.

A further aspect of the present disclosure provides a method for the treatment of a condition caused by, associated with or resulting in increased sensitivity to nerve growth factor (NGF) in an equine subject, said method comprising the steps of:
- providing a therapeutically effective amount of an anti-equine NGF antibody according to the disclosure or an antigen binding fragment thereof, and
- administering the same to an equine in need thereof.

According to a yet further aspect of the present disclosure there is provided a method for the treatment of a tumour induced to proliferate by NGF in an equine and conditions associated therewith, said method comprising the steps of:
- providing a therapeutically effective amount of an anti-equine NGF antibody according to the disclosure or antigen binding fragment thereof, and
- administering the same to an equine in need thereof.

In certain instances, the tumour is an osteosarcoma. In certain instances, the tumour is induced to proliferate by autocrine or paracrine NGF.

In certain instances, the foregoing methods of the disclosure further comprise the step of co-administering at least one further agent which may enhance and/or complement the effectiveness of the anti-NGF antibody of the disclosure. For example, the antibody or antigen binding fragment thereof may be co-administered along with at least one analgesic, NSAID, opioid, corticosteroid, steroid, hyaluronan or hyaluronic acid.

Examples of suitable analgesics include, but are not limited to butorphanol, buprenorphine, fentanyl, flunixin meglumine, merpidine, morphine, nalbuphine and derivatives thereof. Suitable NSAIDS include, but are not limited to, acetaminophen, acetylsalicylic acid, carprofen, etodolac, ketoprofen, meloxicam, firocoxib, robenacoxib, deracoxib and the like.

In certain further instances, the at least one further agent may be a therapeutically active agent which may be one or more of the group selected from: an antibiotic, antifungal, antiprotozoal, antiviral or similar therapeutic agents. Furthermore the at least one further agent may be an inhibitor of mediator(s) of inflammation such as a PGE-receptor antagonist, an immunosuppressive agent, such as cyclosporine, an anti-inflammatory glucocorticoids. In certain further aspects the at least one further agent may be an agent which is used for the treatment of cognitive dysfunction or impairment, such as memory loss or related conditions which may become increasingly prevalent in older equines. Further still, the at least one further agent may be an anti-hypertensive or other compound used for the treatment of cardiovascular dysfunction, for example to treat hypertension, myocardial ischemia, congestive heart failure and the like. Further still, the at least one further agent may be a diuretic, vasodilator, beta-adrenergic receptor antagonist, angiotensin-II converting enzyme inhibitor, calcium channel blocker, HMG-CoA reductase inhibitor, phenylbutazone, hyaluronic acid, polysulphated glycosaminoglycan, interleukin-1 receptor antagonist, IRAP, diclofenac and disease modifying osteoarthritic drugs.

In certain instances, the antibody or antigen binding fragment is administered to the equine as part of the foregoing methods at a dose ranging from about 0.01 mg/kg of body weight to about 10 mg/kg of body weight, in particular from 0.03 mg/kg of body weight to about 3 mg/kg of body weight.

In various further aspects, the present disclosure extends to a composition comprising an antibody or binding fragment thereof according to any foregoing aspect of the disclosure. In certain instances, the composition further comprises at least one pharmaceutically acceptable carrier.

A yet further aspect of the disclosure provides a pharmaceutical composition for treating pain, or a condition resulting in or caused by chronic pain in an equine, comprising a pharmaceutically effective amount of an anti-equine NGF equinised antibody according to the present disclosure, along with at least one pharmaceutically acceptable carrier, excipient or diluent. In certain instances, the composition may further comprise at least one analgesic, NSAID, opioid, corticosteroid or steroid.

In various further aspects, the present disclosure extends to isolated nucleic acid which encodes the antibody or antibody binding fragments of the disclosure.

Accordingly, a yet further aspect of the disclosure provides an isolated nucleic acid that encodes an antibody or antigen binding fragment according to any of the foregoing aspects of the disclosure. In certain instances, the polynucleotide encodes the a light chain variable domain of an anti-equine NGF equinised antibody or antibody fragment having the amino acid sequence of SEQ ID NO:1 or a light chain having the amino acid sequence of SEQ ID NO:4.

In certain further instances the polynucleotide encodes a heavy chain variable domain of an anti-equine NGF equinised antibody or antibody fragment having the amino acid sequence of SEQ ID NO:2 or a heavy chain having the amino acid sequence of SEQ ID NO:5, SEQ ID NO:6, SEQ ID NO:7, SEQ ID NO:8 or SEQ ID NO:9.

In certain instances, the isolated nucleic acid further comprises a nucleic acid encoding one or more regulatory sequences operably linked thereto.

In a further aspect there is provided an expression vector comprising a polynucleotide comprising a polynucleotide encoding a heavy and/or light chain variable domain or a heavy and/or light chain constant domain of the disclosure.
In certain instances the expression vector further comprises one or more regulatory sequences. In certain instances the vector is a plasmid or a retroviral vector.

A yet further aspect provides a host cell incorporating the expression vector of the foregoing aspect of the disclosure. A further aspect of the disclosure provides a host cell which produces the antibody of any of the foregoing aspects of the disclosure.

A yet further aspect of the disclosure provides a method for producing an equinised anti-equine NGF neutralising antibody, the method comprising the step of culturing the host cell of the foregoing aspect of the disclosure to allow the cell to express the equinised anti-equine NGF neutralising antibody.

A yet further aspect of the present disclosure provides a method of producing an anti-equine NGF equinised antibody according to the disclosure comprising the steps of expressing one or more of the polynucleotides / nucleic acids or vectors of the foregoing aspects of the disclosure which express the light and/or heavy chains of the antibodies of the disclosure in a suitable host cell, recovering the expressed polypeptides, which may be expressed together in a host cell, or separately in different host cells, and isolating antibodies.

A yet further aspect of the disclosure provides a method for treating, ameliorating or inhibiting pain in an equine, the method comprising the step of administering to the equine an effective amount of a polynucleotide according to any of the foregoing aspects of the disclosure.

A yet further aspect of the disclosure provides an antibody or antibody binding fragment according to any of the foregoing aspects of the disclosure, or a pharmaceutical composition according to the foregoing aspects of the disclosure, or a nucleic acid according to the foregoing aspects of the disclosure, or a vector according to any of the foregoing aspects of the disclosure for use in the treatment, prevention or amelioration of pain in an equine.

In certain instances, the pain is acute pain. In further instances the pain is chronic pain. Furthermore, the pain may be post-operative pain, or pain resulting from any operating procedure which in equines may include, but is not limited to, orthopaedic surgery, soft tissue surgery, ovariohysterectomy procedures and the like. In certain further instances, the pain is chronic pain associated with cancer or a cancerous condition. In certain further instances, the pain is associated with, or resulting from, inflammation, pruritis, rheumatoid arthritis or osteoarthritis. The pain can be associated with, or resulting from, palmar foot pain, subsolar bruising, laminitis, hoof abscesses, post showing trauma, post race trauma, navicular syndrome and proximal suspensory desmitis

A yet further aspect of the disclosure provides an antibody or antibody binding fragment according to any of the foregoing aspects of the disclosure, or a pharmaceutical composition according to the foregoing aspects of the disclosure, or a nucleic acid according to the foregoing aspects of the disclosure, or a vector comprising the same according to any of the foregoing aspects of the disclosure for use in the treatment or osteoarthritis and/or rheumatoid arthritis.

A yet further aspect of the disclosure provides an antibody or antibody binding fragment according to any of the foregoing aspects of the disclosure, or a pharmaceutical composition according to the foregoing aspects of the disclosure, or a nucleic acid or vector comprising the same according to any of the foregoing aspects of the disclosure for use in the treatment of a tumour induced to proliferate by NGF in an equine and conditions associated therewith, in particular osteosarcoma. In certain instances, the tumour is induced to proliferate by autocrine or paracrine NGF.

A yet further aspect of the disclosure provides use of an antibody or antibody binding fragment according to any of the foregoing aspects of the disclosure, or a pharmaceutical composition according to the foregoing aspects of the disclosure, or a nucleic acid according to the foregoing aspects of the disclosure, or a vector comprising the same according to any of the foregoing aspects of the disclosure in the preparation of a medicament for the treatment or prevention of pain in an equine.

The pain may be acute or chronic pain. In certain instances the pain is chronic pain. Furthermore, the pain may be post-operative pain, or pain resulting from any operating procedure which in equines may include, but is not limited to, orthopaedic surgery, soft tissue surgery and the like. In certain further instances, the pain is chronic pain associated with cancer or a cancerous condition. In certain further instances, the pain is associated with, or resulting from, inflammation, pruritis, rheumatoid arthritis or osteoarthritis or further can be pain associated with, or resulting from, palmar foot pain, subsolar bruising, laminitis, hoof abscesses, post showing trauma, post race trauma, navicular syndrome and proximal suspensory desmitis

A yet further aspect of the disclosure provides use of an antibody or antibody binding fragment according to any of the foregoing aspects of the disclosure, or a pharmaceutical composition according to the foregoing aspects of the disclosure, or a nucleic acid according to the foregoing aspects of the disclosure, or a vector comprising the same according to any of the foregoing aspects of the disclosure in the preparation of a medicament for the treatment, inhibition amelioration or prevention of rheumatoid arthritis or osteoarthritis in an equine.

A yet further aspect of the disclosure provides use of an antibody or antibody binding fragment according to any of the foregoing aspects of the disclosure, or a pharmaceutical composition according to the foregoing aspects of the disclosure, or a nucleic acid or vector comprising the same according to any of the foregoing aspects of the disclosure in the preparation of a medicament for the treatment of a tumour induced to proliferate by NGF in an equine and conditions associated therewith, in particular osteosarcoma. In certain instances, the tumour is induced to proliferate by autocrine or paracrine NGF.

In a yet further aspect there is provided a cell line, or a derivative or progeny cell thereof, that produces anti-equine NGF neutralising monoclonal antibodies, or fragments thereof according to the disclosure.

A yet further aspect of the present disclosure provides a kit for the treatment of pain in equines, or for the treatment of a condition associated with pain, or for the treatment, amelioration or inhibition of pain associated with osteoarthritis, rheumatoid arthritis, inflammation, pruritis, palmar foot pain, subsolar bruising, laminitis, hoof abscesses, post showing trauma, post race trauma, navicular syndrome and proximal suspensory desmitis comprising an anti-equine NGF antibody according to any of the foregoing aspects of the disclosure and instructions for use of the same.

A yet further aspect of the present disclosure provides a diagnostic kit for the detection of an anti-equine NGF monoclonal antibody in fluids in vitro, ex vivo and in vivo, for use in determining the concentration of said antibody. The kit may comprise any of the antibodies of the disclosure or a binding fragment thereof. The kit may comprise instructions for use of same.

### Brief Description of the Figures

Figure 1 is a graph showing the binding of an equinised antibody produced according to the invention to murine and equine NGF.
Figure 2A and B show a gel showing protein A purification of the equinised antibodies prepared by the method of the invention as revealed by Western blotting using anti-equine polyclonal antibody specific to the heavy chain (A) and a gel showing the results of purification of equinised antibodies using SDS-Page (B).
Figure 3 shows a graph showing the inhibition of NGF induced proliferation of TF-1 cells by equinised antibodies.
Figure 4 shows a graph showing a lack of complement deposition induced by antigen-captured equinised antibodies.
Figure 5 shows the amino acid sequence of a light chain variable domain of the equinised anti-NGF (SEQ ID NO:1). The three CDR regions, identified according to Kabat numbering, are underlined. Asterisks above a specific residue indicate differences in the sequence between the rat aD11 anti-murine NGF monoclonal antibody.
Figure 6 shows the amino acid sequence of a heavy chain variable domain of the equinised anti-NGF (SEQ ID NO:2). The three CDR regions, identified according to Kabat numbering, are underlined. Asterisks above a specific residue indicate differences in the sequence between the rat aD11 anti-murine NGF monoclonal antibody.
Figure 7 shows the amino acid sequence (SEQ ID NO:4) of an equinised anti-NGF light chain variable domain equine kappa light chain (eqN-kLC) antibody. Variable domain residues are shown in bold.

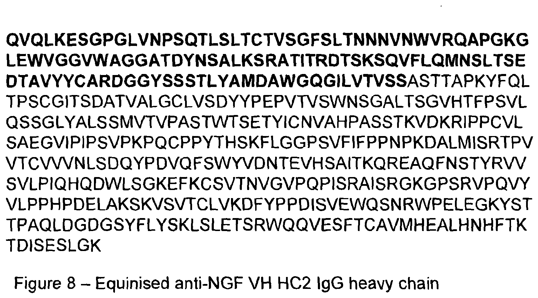

Figure 8 shows the amino acid sequence (SEQ ID NO:6) of an equinised anti-NGF heavy chain variable domain equine IgG-2 heavy chain (eqN-HC2 (IgG2)). Variable domain residues are shown in bold.

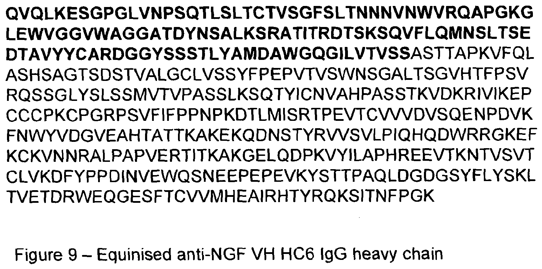

Figure 9 shows the amino acid sequence (SEQ ID NO:7) of an equinised anti-NGF heavy chain variable domain equine IgG-6 heavy chain (eqN-HC6 (IgG2)) having the HC6 heavy chain constant domains. Variable domain residues are shown in bold.

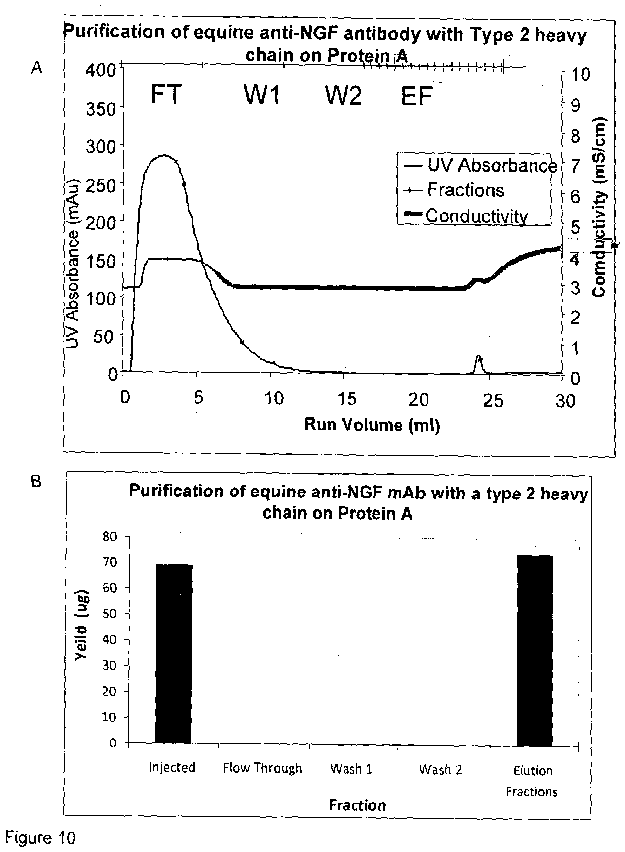

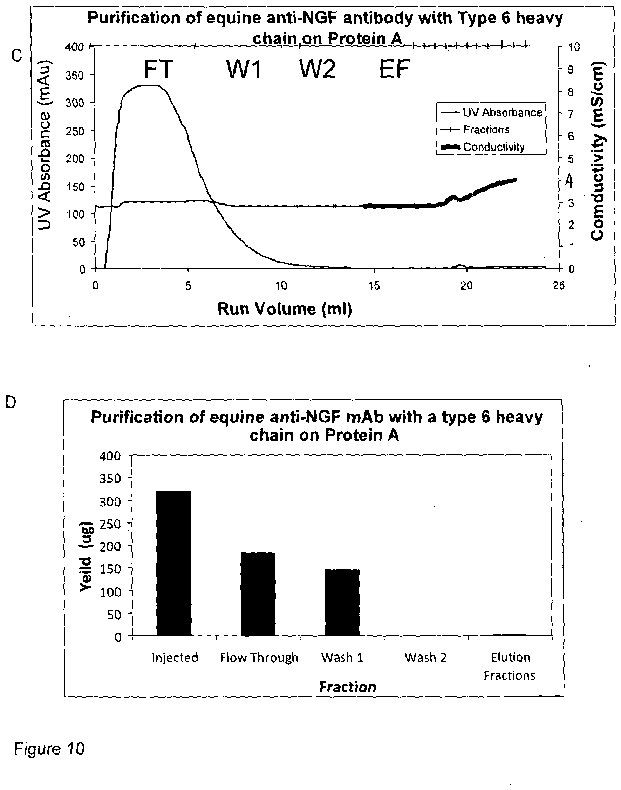

Figure 10 shows a comparison of Protein A affinity chromatography profiles of HC2 and HC6 isotype variants of equinised anti-NGF MAbs. Figure 10A and C illustrate the UV absorbance (dark line) and conductivity profiles (grey line) following loading CHO transfectant supernatants of Type 2 (HC2, Figure 10A) and type 6 (HC6, Figure 10C) antibodies. Figure 10B and D illustrate the recovery of antibody from the column (measured by quantitative ELISA) and show that virtually all of the HC2 antibody bound to the column and was recovered by specific elution (Figure 10B), whereas none of the HC6 antibody was bound by the column (Figure 10D).
Figure 11 shows that anti-canine NGF monoclonal antibodies prepared by a method corresponding to the method of the present invention reduce inflammatory pain in dogs.

### Detailed description of the Invention

Following extensive experimentation, the inventor has taken the rat anti-mouse NGF monoclonal antibody (MAb) αD11 amino acid sequence and used this to produce a non-immunogenic anti-NGF antibody. The resulting antibody, which may be a chimeric or equinised antibody, is not produced using standard CDR grafting techniques and is surprisingly shown to exhibit high affinity binding to equine NGF. Even more surprisingly, the antibody is shown to neutralise equine NGF biological function, most specifically by inhibiting the binding of NGF to cell based receptors TrkA and p75. Furthermore, it has also been discovered, unexpectedly, that when administered to an equine, neutralising antibodies are not produced there against. Accordingly, the non-immunogenic antibody prepared by the method of the invention is suitable for long term relief of chronic pain in horses. The process of generating the heavy and light chain variable domains for the antibodies prepared by the method of the invention which has been employed by the inventor results in the replacement of specific rat (donor) amino acid residues which are present within the framework regions of the light and heavy chain variable domains with residues which, based on the inventor's analysis, will retain the conformation of the CDR regions and therefore maintain binding specificity and avidity, while reducing the presence of immunogenic epitopes which may result in neutralising antibodies being generated against the antibody, if it were to be administered to equines in an unaltered form. Specifically, the method of preparing antibodies of the invention (known as PETisation) comprises assessing the sequence of the framework regions of a donor (e.g. rat) antibody for suitability for administering to a equine by comparing the sequence of the framework regions of the donor antibody with the sequence of an antibody or a pool of antibodies derived from equines. Although the comparison may be between the donor sequence and a single member of the target sequence, it will be obvious that comparison with a pool of target sequences is preferred because this will expand the number of natural options at each Kabat position in the target species. Not only will this increase the chance of a "match" between the donor and the target, but it will also expand the options for replacement where a match does not exist. As a result, a replacement with characteristics as close as possible to the donor will be able to be chosen. Where the donor sequence and the equine sequence differ at any Kabat number or corresponding position, the donor sequence is modified to substitute the amino acid residue in question with an amino acid residue which is known to be natural at that position in equines.
Where substitution of an amino acid residue present in a donor immunoglobulin framework region is required, this is undertaken using the principle of conservative substitution wherein an amino acid residue is replaced with an amino acid residue which is natural at that Kabat position in an equine and is as closely related as possible in size, charge and hydrophobicity to the amino acid being substituted in the donor sequence. The intention is to choose a replacement which would cause no, or at least only minimum, perturbation or disruption to the three-dimensional structure of the donor antibody. In certain situations, there will be no clear option and each choice will have benefits and downsides. A final decision may require three-dimensional modelling or even expression of various alternative sequences. However, generally, a clear preference will be available. As a result of this procedure, a change in the donor sequence is only made when that residue would be foreign in the target and the replacement amino acid is as closely related as possible to that which it replaces. Thus, the creation of foreign epitopes is avoided, but the overall three-dimensional structure is preserved and as a result, affinity and specificity are also preserved.

The light and heavy chain constant regions are typically derived from equine (target) derived antibodies. The heavy chain constant domains are selected or modified such that they do not mediate downstream effector functions. As it has been found, quite surprisingly, that no or minimal neutralising antibodies are produced against the antibodies produced according to the invention, the antibodies have surprisingly been found to have the associated benefit of long circulatory half life and the option for repeat dosing. Furthermore, as the substitution of the framework residues is performed in such a manner that it does not affect the three dimensional conformation of the CDR regions, there will be no variation in binding specificity.

While hybrid murine-equine chimeric antibodies are known, there are currently no examples of fully equinised monoclonal antibodies described in the literature. Accordingly, it is highly unexpected that such an antibody can be produced and shown to have therapeutic utility.

There are four major IgG isotypes in man and mouse and while nomenclature is similar they differ in behaviour and function including affinity for bacterial products such as Protein A and Protein G, their ability to activate the complement dependent cytolysis (CDC) and their ability to induce killing of target cells through antibody dependent cellular cytotoxity (ADCC). The selection of IgG isotypes with CDC and ADCC active or "armed" constant domains is considered to be of clinical benefit when antibodies are designed to eliminate target cells bearing their cognate antigen, such as in oncology or infection control (e.g. in human medical use human IgG1 isotypes are preferred for the above purposes). By contrast, the activation of the immune system is considered undesirable in other settings such as in the relief of inflammation, pain or autoimmunity and so human IgG isotypes with minimal CDC and ADCC activity are preferred (e.g. in such human medical use, IgG4 isotypes are often preferred). Seven distinct immunoglobulin gamma (IgG) heavy chain constant domain isotypes have been described in the equine immune system along with single kappa and lambda constant domain sequences. The seven equine heavy chain constant domains IgG1, IgG2, IgG3, IgG4, IgG5, IgG6 and IgG7 have been characterised in terms of functional activity mediated thereby. The selection of IgG isotypes with CDC and ADCC active constant domains is considered to be of benefit when antibodies are designed to eliminate target cells bearing the cognate antigen, such as in oncology or infection control, e.g. in human medical use human IgG1 isotypes are preferred. By contrast, the activation of the immune system is considered undesirable in other settings such as in the relief of inflammation, pain or autoimmunity and so human IgG isotypes with minimal or "disarmed" CDC and ADCC activity are preferred, e.g. in human medical use, IgG4 isotypes would be selected. Equine MAb isotypes have a broader spectrum of activities and so the selection of armed or disarmed heavy chains is presumed to be of similar value.

The antibodies of the disclosure comprise equine derived heavy and light chain constant domains. Furthermore, the complementarity determining regions are derived from the rat αD11 anti-mouse NGF antibody. The αD11 antibody was first described by Cattaneo et al. (Cattaneo A, Rapposelli B, Calissano P. (1988) "Three distinct types of monoclonal antibodies after long-term immunization of rats with mouse nerve growth factor". J Neurochem 50(4):1003-1010). The alphaD11 antibody was subsequently cloned by Ruberti et al. (Ruberti, F. et al. (1993) "Cloning and Expression of an Anti-Nerve Growth Factor (NGF) Antibody for Studies Using the Neuroantibody Approach". Cellular and Molecular Neurobiology. 13(5):559-568).

The CDR regions derived from the αD11 antibody are combined with framework region sequences which have been determined by the inventor to preserve CDR tertiary structure, and therefore binding specificity, while preventing neutralising antibodies being raised there against, when the antibody is administered to an equine.

Each of the light and heavy chain variable regions contains four framework regions, referred to as FR1-FR4. For each of these framework regions, the inventor has identified a preferred amino residue (a so called preferred residue) for each specific position, and furthermore alternative amino acid residues which could also be provided at that position. Tables 1 to 8 below illustrate the 4 framework regions for each of the heavy and light chains. The tables provide the amino acid position relative to that specific framework region and further according to the Kabat numbering system used to identify the position of a particular residue along the length of the complete heavy or light chain variable domain. The residue or residues shown as group 1 residues are the preferred residues, while the group 2 residues are alternative residues. However these would generally not be preferable to the residues shown in group 1 relating to that specific position. The amino acid residues are identified using the single letter nomenclature system.

**Table 1 - Light chain variable domain FR1 residues**

| Light chain FR1 position | Kabat light chain numbering | Group 1 amino acid residues | Group 2 amino acid residues |
|---|---|---|---|
| 1 | 1 | D | GKV |
| 2 | 2 | IV | FNST |
| 3 | 3 | V | AGIM |
| 4 | 4 | M | LQV |
| 5 | 5 | T | AI |
| 6 | 6 | Q | |
| 7 | 7 | ST | F |
| 8 | 8 | P | |
| 9 | 9 | AE | DPS |
| 10 | 10 | S | FLT |
| 11 | 11 | LV | S |
| 12 | 12 | STA | EV |
| 13 | 13 | AV | LQT |
| 14 | 14 | ST | AP |
| 15 | 15 | L | PR |
| 16 | 16 | G | R |
| 17 | 17 | EQ | |
| 18 | 18 | TR | SGK |
| 19 | 19 | V | A |
| 20 | 20 | ET | DV |
| 21 | 21 | ILMV | T |
| 22 | 22 | EK | LNQRST |
| 23 | 23 | C | |

**Table 2 - Light chain variable domain FR2 residues**

| Light chain FR2 position | Kabat light chain numbering | Group 1 amino acid residues | Group 2 amino acid residues |
|---|---|---|---|
| 1 | 35 | W | |
| 2 | 36 | Y | FH |
| 3 | 37 | Q | RS |
| 4 | 38 | Q | HKRV |
| 5 | 39 | KR | V |
| 6 | 40 | P | ILS |
| 7 | 41 | G | |
| 8 | 42 | QE | |
| 9 | 43 | AS | PRVT |
| 10 | 44 | P | L |
| 11 | 45 | KRE | IL |
| 12 | 46 | LR | AEGHQW |
| 13 | 47 | L | FIMV |
| 14 | 48 | I | FTMV |
| 15 | 49 | Y | ACDEFG HQRSTV |

**Table 3 - Light chain variable domain FR3 residues**

| Light chain FR3 position | Kabat light chain numbering | Group 1 amino acid residues | Group 2 amino acid residues |
|---|---|---|---|
| 1 | 57 | G | DF |
| 2 | 58 | V | AF |
| 3 | 59 | P | LS |
| 4 | 60 | SD | AEGL |
| 5 | 61 | R | |
| 6 | 62 | FY | L |
| 7 | 63 | S | CFGNRT |
| 8 | 64 | G | A |
| 9 | 65 | S | DEGKRTW |
| 10 | 66 | G | ARV |
| 11 | 67 | S | AFTY |
| 12 | 68 | G | ET |
| 13 | 69 | T | ASW |
| 14 | 70 | DE | |
| 15 | 71 | YF | |
| 16 | 72 | ST | AV |
| 17 | 73 | L | FP |
| 18 | 74 | T | AISV |
| 19 | 75 | I | V |
| 20 | 76 | NS | DGT |
| 21 | 77 | S | DEPRT |
| 22 | 78 | L | |
| 23 | 79 | Q | ER |
| 24 | 80 | AS | ET |
| 25 | 81 | E | ADGT |
| 26 | 82 | D | N |
| 27 | 82A | V | ALEGS |
| 28 | 82B | A | G |
| 29 | 82C | IST | DEFLMNV |
| 30 | 83 | Y | C |
| 31 | 84 | FY | HSTVW |
| 32 | 85 | C | |

**Table 4 - Light chain variable domain FR4 residues**

| Light chain FR4 position | Kabat light chain numbering | Group 1 amino acid residues | Group 2 amino acid residues |
|---|---|---|---|
| 1 | 95 | F | IL |
| 2 | 96 | G | |
| 3 | 97 | Q | L |
| 4 | 98 | G | |
| 5 | 99 | T | S |
| 6 | 100 | K | MNR |
| 7 | 101 | L | MV |
| 8 | 102 | E | ADK |
| 9 | 103 | IL | FMV |
| 10 | 104 | K | AEGIQRTV |

**Table 5 - Heavy chain variable domain FR1 residues**

| Heavy chain FR1 position | Kabat heavy chain numbering | Group 1 amino acid residues | Group 2 amino acid residues |
|---|---|---|---|
| 1 | 1 | Q | |
| 2 | 2 | V | |
| 3 | 3 | Q | |
| 4 | 4 | L | |
| 5 | 5 | K | Q |
| 6 | 6 | E | |
| 7 | 7 | S | |
| 8 | 8 | G | |
| 9 | 9 | P | |
| 10 | 10 | G | D |
| 11 | 11 | L | Q |
| 12 | 12 | V | M |
| 13 | 13 | NK | MR |
| 14 | 14 | P | IS |
| 15 | 15 | S | AG |
| 16 | 16 | Q | E |
| 17 | 17 | T | A |
| 18 | 18 | L | |
| 19 | 19 | S | T |
| 20 | 20 | L | |
| 21 | 21 | T | SV |
| 22 | 22 | C | |
| 23 | 23 | T | AFS |
| 24 | 24 | V | I |
| 25 | 25 | S | T |
| 26 | 26 | G | A |
| 27 | 27 | FL | AGIMNQS |
| 28 | 28 | S | DHILNP |
| 29 | 29 | L | DSTV |
| 30 | 30 | TS | EINR |

**Table 6 - Heavy chain variable domain FR2 residues**

| Heavy Chain FR2 position | Kabat heavy chain numbering | Group 1 Amino Acid residues | Group 2 Amino Acid residues |
|---|---|---|---|
| 1 | 36 | W | |
| 2 | 37 | V | L |
| 3 | 38 | R | |
| 4 | 39 | Q | |
| 5 | 40 | A | PSV |
| 6 | 41 | P | |
| 7 | 42 | G | |
| 8 | 43 | K | W |
| 9 | 44 | G | R |
| 10 | 45 | L | PW |
| 11 | 46 | E | |
| 12 | 47 | WF | EHRVY |
| 13 | 48 | V | |
| 14 | 49 | G | ADS |

**Table 7 - Heavy chain variable domain FR3 residues**

| Heavy chain FR3 position | Kabat heavy chain numbering | Group 1 amino acid residues | Group 2 amino acid residues |
|---|---|---|---|
| 1 | 66 | R | |
| 2 | 67 | A | CGITV |
| 3 | 68 | ST | DIMNR |
| 4 | 69 | I | V |
| 5 | 70 | T | ILS |
| 6 | 71 | RK | ES |
| 7 | 72 | D | EN |
| 8 | 73 | T | AEIPSY |
| 9 | 74 | S | EGKT |
| 10 | 75 | K | ELNQR |
| 11 | 76 | S | GKNR |
| 12 | 77 | Q | EHR |
| 13 | 78 | V | AILFS |
| 14 | 79 | FY | LRSTV |
| 15 | 80 | L | V |
| 16 | 81 | QT | I |
| 17 | 82 | ML | V |
| 18 | 82A | N | DKRST |
| 19 | 82B | S | DEGKMT |
| 20 | 82C | L | MV |
| 21 | 83 | T | S |
| 22 | 84 | S | DEGR |
| 23 | 85 | E | DG |
| 24 | 86 | D | |
| 25 | 87 | T | A |
| 26 | 88 | A | S |
| 27 | 89 | V | D |
| 28 | 90 | Y | |
| 29 | 91 | Y | AFIW |
| 30 | 92 | C | |
| 31 | 93 | A | EGISTV |
| 32 | 94 | RG | AEGHIKS |

**Table 8 - Heavy chain variable domain FR4 residues**

| Heavy Chain FR4 position | Kabat heavy chain numbering | Group 1 Amino Acid residues | Group 2 Amino Acid residues |
|---|---|---|---|
| 1 | 103 | W | |
| 2 | 104 | G | |
| 3 | 105 | Q | P |
| 4 | 106 | G | |
| 5 | 107 | I | |
| 6 | 108 | L | |
| 7 | 109 | V | |
| 8 | 110 | T | |
| 9 | 111 | V | |
| 10 | 112 | S | |
| 11 | 113 | - | |

The equinised antibody of the disclosure therefore differs from, for example, a chimeric monoclonal antibody which consists of a complete variable region derived from a first species and constant domains derived from a second species, or from a CDR-grafted equinised antibody, where the complementarity determining regions (CDRs) of the heavy and light chain variable regions comprise amino acid residues derived from a donor antibody and introduced into framework regions (FR) and constant regions (CR) derived from a target antibody or from equine germline sequences.

It is preferred that the equinised antibody substantially retains the binding properties of the parent (donor) antibody from which the CDRs are derived. That means that the equinised antibody will exhibit the same or substantially the same antigen-binding affinity and avidity as the donor antibody from which the CDRs are derived. Ideally, the affinity of the equinised antibody will not be less than 10% of the donor antibody affinity for the target epitope, more preferably not less than about 30%, and most preferably the affinity will not be less than 50% of the parent (donor) antibody. Methods for assaying antigen-binding affinity are well known in the art and include half-maximal binding assays, competition assays, and Scatchard analysis.

As defined hereinbefore, the present disclosure extends to binding members or antigen binding fragments derived from the equinised antibodies of the disclosure. Such antigen binding fragments refer to one or more fragments of an antibody that retain the ability to specifically bind to equine NGF. It has been shown that the antigen binding function of an antibody can be performed by fragments of a full length antibody. In certain instances, the binding members or antigen binding fragments may be isolated binding members. A binding member or antigen binding fragment of the disclosure may comprise a fragment of the antibodies of the present disclosure, e.g. a fragment of a fully equinised antibody molecule, such as the heavy or light chain only, or, for example, the variable domain of the heavy and/or light chain. In certain instances, a binding member may typically comprise, consist, or consist essentially of an antibody VH and/or VL domain. VH domains of binding members are also provided as part of the disclosure. Within each of the VH and VL domains are 3 complementarity determining regions ("CDRs"), along with 4 associated framework regions ("FRs"). A VH domain typically comprises 3 HCDRs (heavy chain complementarity determining regions), and a VL domain typically comprises 3 LCDRs (light chain complementarity regions). Accordingly, a binding member may comprise a VH domain comprising, in sequence, VH CDR1 (or HCDR1), CDR2 (HCDR2) and CDR3 (HCDR3) regions along with a plurality of associated framework regions. A binding member may additionally or alternatively comprise a VL domain comprising VL CDR1, CDR2 and CDR3 domains along with associated framework regions. The VH or VL domains typically comprise four framework regions, FR1, FR2, FR3 and FR4, interspersed with the 3 complementarity determining regions in the following arrangement: FR1-CDR1-FR2-CDR2-FR3-CDR3-FR4.

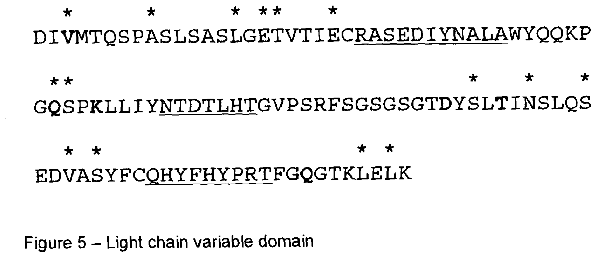

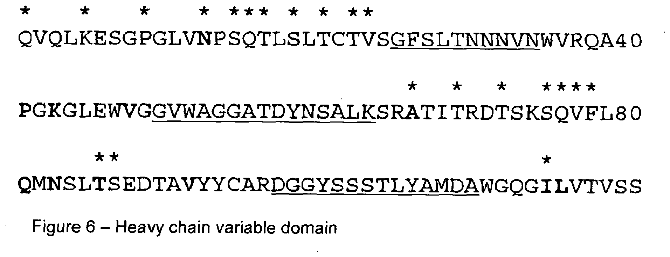

Figure 5 shows the amino acid sequence of a light chain variable domain of an anti-NGF antibody prepared according to the invention. The CDR1, CDR2 and CDR3 regions are underlined. Further, Figure 6 shows the amino acid sequence of a heavy chain variable domain of an anti-NGF antibody prepared according to the invention. The CDR1, CDR2 and CDR3 regions are underlined.

In Figures 5 and 6, the residues of the light chain variable domain (Figure 5) and heavy chain variable domain (Figure 6) can conventionally numbered according to the numbering system devised by Kabat et al. (Kabat,E.A., Wu,T.T., Perry,H., Gottesman,K. and Foeller,C. (1991) Sequences of Proteins of Immunological Interest, Fifth Edition. NIH Publication No. 91-3242, Kabat et al. (1971) Ann. NY Acad, Sci. 190:382-391). The Kabat numbering system refers to a system of numbering amino acid residues which are more variable (i.e. hypervariable) than other amino acid residues in the heavy and light chain variable regions of an antibody, or an antigen binding portion thereof). The Kabat numbering system is generally used when referring to a residue in the variable domain (approximately residues 1-104 of the light chain and residues 1-113 of the heavy chain). This numbering system is only used in the present specification where specifically stated. This is because the Kabat residue designations do not always correspond directly with the linear numbering of the amino acid residues of the heavy and light chain variable regions of the present disclosure provided in the relevant sequences listed herein. In particular, the actual linear amino acid sequence may contain fewer or additional amino acids than in the strict Kabat numbering corresponding to a shortening of, or insertion into, a structural component, whether a framework region or complementarity determining region (CDR), of the basic variable domain structure of the heavy or light chain. The correct Kabat numbering of residues may be determined for any given antibody by alignment of residues in the sequence of the antibody with a standard sequence to which the Kabat numbering has been applied.

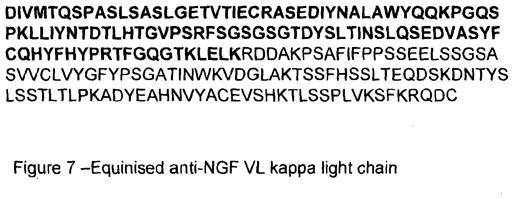

Figure 6 shows a heavy chain variable domain amino sequence. This is also shown in SEQ ID NO:2. However, in Figure 6, the numbering takes account of amino acid residues 80, 80A, 80B, and 80C these being Kabat numbering provisions, whereas in SEQ ID NO:2, the linear numbering continues sequentially, that residues 80, 80A, 80B, and 80C are listed sequentially as 80, 81, 82 and 83. The same is true for Kabat residues 100, 100A, 100B, 100C, 100D, 100E and 100F in Figure 7.

As described hereinbefore, an antibody binding fragment may be selected from the group comprising, but not limited to, a Fab fragment, a Fab' fragment and a scFv (single chain variable fragment), or from a peptidomimetic, a diabody, or a related multivalent derivative.

In certain instances the antibody binding fragment is a Fab or F(ab')2 fragment, which consists of the VL, VH, CL and CH1 domains of a heterotetrameric antibody. In certain instances, the VL domain has an amino acid sequence of SEQ ID NO:1 and the VH domain has an amino acid sequence of SEQ ID NO:2. In certain instances, the CL and CH1 domains are based on the amino acid sequence of a CL and CH1 domain of an equine immunoglobulin, in particular an IgG2 (HC2) or IgG6(HC6) equine derived constant domain.

Techniques used for the recombinant production of Fab, Fab' and F(ab')2 fragments are well known to the person skilled in the art and include those disclosed in International PCT Patent Publication WO 92/22324, and in Sawai et al., "Direct Production of the Fab Fragment Derived From the Sperm Immobilizing Antibody Using Polymerase Chain Reaction and cDNA Expression Vectors", 1995, AJRI 34:26-34. Examples of techniques which can be used to produce scFv (single chain Fv fragments) are disclosed in Huston et al., "Protein Engineering of Single-Chain Fv Analogs and Fusion Proteins", Methods in Enzymology, vol. 203:46-88 (1991).

In certain instances, antibody fragments can be derived from full length antibodies by proteolytic digestion according to the method of Morimoto (Morimoto et al., "Single-step purification of F(ab')2 fragments of mouse monoclonal antibodies (immunoglobulins G1) by hydrophobic interaction high performance liquid chromatography using TSKgel Phenyl-5PW" Journal of Biochemical and Biophysical Methods 24:107-117 (1992)). Antibody fragments can also be produced directly by host cells (Carter et al., "High level Escherichia coli expression and production of a bivalent humanized antibody fragment" Bio/Technology 10:163-167 (1992)).

In addition to providing an equinised monoclonal antibody which has binding specificity to equine NGF and which antagonises equine NGF function, the present disclosure further extends to binding members other than antibodies comprising a pair of binding domains based on the amino acid sequence of a VL (light chain variable) region as defined in SEQ ID NO:1 and an amino acid sequence of a VH (heavy chain variable) region as defined in SEQ ID NO:2. In particular, the disclosure extends to single binding domains which are based on either the VL or VH region of the equinised antibodies of the antibodies of the disclosure.

Accordingly, in certain further instances of the present disclosure, there is provided a binding member comprising, consisting or consisting essentially of a single binding domain derived from the humanised antibody of the disclosure. In certain instances, the single binding domain is derived from the amino acid sequence of the VH (heavy chain variable domain) as defined in SEQ ID NO:2 or SEQ ID NO:4. Such a binding domain may be used as a targeting agent to equine NGF.

In certain embodiments, further engineering techniques can be used to modify the antibodies of the present disclosure, for example by including modifications of the Fc region which can alter serum half life, complement fixation, Fc receptor binding and/or antigen dependent cellular cytotoxicity. Further, in certain embodiments, antibodies or antibody fragments can be produced which have altered glycosylation patterns. In certain embodiments, an antibody is altered to increase or decrease the extent to which the antibody is glycosylated. Glycosylation of polypeptides is typically either N-linked or O-linked. N-linked refers to the attachment of a carbohydrate moiety to the side chain of an asparagine residue. The tripeptide sequences asparagine-X-serine and asparagine-X -threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N- aceylgalactosamine, galactose, or xylose to a hydroxyamino acid, most commonly serine or threonine, although 5-hydroxyproline or 5-hydroxylysine may also be used. The inventor has provided aglycosylated equine constant domains, these being defined herein as SEQ ID NO:8 or SEQ ID NO:9.

In certain further instances, the anti-equine NGF antibodies of the disclosure can be PEGylated by reacting the antibody with a plyethylene glycol (PEG) derivative. In certain instances, the antibody is defucosylated and therefore lacks fucose residues.

In certain embodiments, modifications in the biological properties of an antibody may be accomplished by selecting substitutions that affect (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (A. L. Lehninger, in Biochemistry, 2nd Ed., 73-75, Worth Publishers, New York (1975)): (1) non-polar: Ala (A), Val (V), Leu (L), Ile (I), Pro (P), Phe (F), Trp (W), Met (M); (2) uncharged polar: Gly (G), Ser (S), Thr (T), Cys (C), Tyr (Y), Asn (N), Gln (Q); (3) acidic: Asp (D), Glu (E); (4) basic: Lys (K), Arg (R), His(H). Alternatively, naturally occurring residues may be divided into groups based on common side-chain properties: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; (6) aromatic: Trp, Tyr, Phe. Non-conservative substitutions will entail exchanging a member of one of these classes for another class. Such substituted residues also may be introduced into the conservative substitution sites or, into the remaining (e.g. non-conserved) sites.

In various further aspects, the present disclosure extends to an immunoconjugate comprising an anti-equine NGF antibody of the disclosure, or an antigen binding portion thereof linked to a partner molecule. In certain instances, such an antibody-partner molecule conjugate is conjugated by means of a chemical linker, such as a peptidyl linker, a hydrazine linker or a disulphide linker. In certain instances, the coupling partner is an effector molecule, label, drug, or carrier molecule. Suitable techniques for coupling the antibodies of the disclosure to both peptidyl and non-peptidyl coupling partners will be well known to persons skilled in the art. Examples of suitable labels include detectable labels, such as a radiolabel, or an enzymatic label, such as horse radish peroxidase, or chemical moieties, such as biotin. Alternatively, the label may be a functional label, for example, ricin, or pro-drugs which are capable of converting prodrugs into active drugs at the site of antibody binding.

In various further aspects, the present disclosure extends to polynucleotides, and in particular isolated polynucleotides, which encode the equinised antibodies, antibody fragments and binding members of the present disclosure. As defined herein, a "polynucleotide" includes any polyribonucleotide or polydeoxyribonucleotide, which may be unmodified RNA or DNA, or modified RNA or DNA, including without limitation, single and double stranded RNA, and RNA which is a mixture of single and double stranded regions. A polynucleotide of the disclosure, e.g. a polynucleotide which encodes a polypeptide or polypeptides of the disclosure includes allelic variants thereof and/or their complements including a polynucleotide that hybridises to such nucleotide sequences under conditions of moderate or high stringency.

The present disclosure further extends to antibody mimetics, such as domain antibodies, nanobodies, unibodies, versabodies, and duocalins which are based on the equine NGF antibodies of the present disclosure. A wide variety of antibody mimetic technologies are known to the person skilled in the art. For example, so called, domain antibodies (Domantis, UK) are small functional binding units of antibodies which correspond to the variable regions of either the light or heavy chains of human antibodies. Directions for the production of such domain antibodies can be found in US Patent No. 6,291,158, US Patent No. 6,582,915 and US Patent No. 6,593,081. Nanobodies are antibody-derived therapeutic proteins which contain unique structural and functional properties of naturally occurring heavy chain antibodies found in camelids. Unibodies are a further antibody fragment technology, based upon the removal of the hinge region of IgG4 antibodies. The deletion of the hinge region results in a molecule which is approximately half the size of a traditional IgG4 antibody and which has a univalent binding region. Unibodies preserve the property of IgG4 antibodies of being inert and therefore not inducing immune responses.

Further binding molecules include affibody molecules (US Patent 5,831,012), DARPins (designed ankyrin repeat proteins) (International PCT Patent Application Publication WO 02/20565) and anticalins (US Patent No. 7,250,297 and WO 99/16873). Verabodies are a further antibody mimetic technology. Versabodies (Amunix, US Patent Application Publication No. 2007/0191272) are small proteins, referred to as microproteins, of 3-5kDa with greater than 15% cysteine residues, which form a high disulphide bond density scaffold which replaces the hydrophobic core which protein typically exhibit

Avimers are another type of antibody mimetic. Avimers originate from the recombination of families of human serum proteins. They are single protein chains composed of modular binding domains, each of which is designed to bind to a particular target site. The avimers can bind simultaneously to sites on a single protein target and/or sites on multiple protein targets. Known as multi-point attachment or avidity, this binding mechanism mimics the way cells and molecules interact in the body, supports the generation of antagonists and agonists, and results in drugs with multiple functions and potent activity. Avimers libraries can be produced according to WO 2004/044011 and for example US 2005/0053973. Avimers libraries are also available commercially from Avidia Inc, Mountain View, California, USA.

### Antibody production

The antibodies and binding members prepared by the method of the invention may be produced wholly or partly by chemical synthesis. For example, the antibodies and binding members can be prepared by techniques which are well known to the person skilled in the art, such as standard liquid peptide synthesis, or by solid-phase peptide synthesis methods. Alternatively, the antibodies and binding members may be prepared in solution using liquid phase peptide synthesis techniques, or further by a combination of solid-phase, liquid phase and solution chemistry.

The present invention further extends to the production of the antibodies or binding members prepared by the method of the invention by expression of a nucleic acid which encodes at least one amino acid which comprises an antibody prepared by the method of the invention in a suitable expression system, such that a desired peptide or polypeptide can be encoded. For example, a nucleic acid encoding the amino acid light chain and a second nucleic acid encoding an amino acid heavy chain can be expressed to provide an antibody prepared by the method of the present invention.

In certain further aspects of the disclosure, there is provided nucleic acids encoding amino acid sequences which form the antibodies or binding members of the present disclosure.

Typically, nucleic acids encoding the amino acid sequences which form antibodies or binding members prepared by the method of the present invention can be provided in an isolated or purified form, or provided in a form which is substantially free of material which can be naturally associated with it, with the exception of one or more regulatory sequences. Nucleic acid which expresses an antibody or binding member prepared by the method of the invention may be wholly or partially synthetic and may include, but is not limited to DNA, cDNA and RNA.

Nucleic acid sequences encoding the antibodies or binding members prepared by the method of the invention can be readily prepared by the skilled person using techniques which are well known to those skilled in the art, such as those described in Sambrook et al. "Molecular Cloning", A laboratory manual, cold Spring Harbor Laboratory Press, Volumes 1-3, 2001 (ISBN-0879695773), and Ausubel et al. Short Protocols in Molecular Biology. John Wiley and Sons, 4th Edition, 1999 (ISBN - 0471250929). Said techniques include (i) the use of the polymerase chain reaction (PCR) to amplify samples of nucleic acid, (ii) chemical synthesis, or (iii) preparation of cDNA sequences. DNA encoding antibodies or binding members prepared by the method of the invention may be generated and used in any suitable way known to those skilled in the art, including taking encoding DNA, identifying suitable restriction enzyme recognition sites either side of the portion to be expressed, and cutting out said portion from the DNA. The excised portion may then be operably linked to a suitable promoter and expressed in a suitable expression system, such as a commercially available expression system. Alternatively, the relevant portions of DNA can be amplified by using suitable PCR primers. Modifications to the DNA sequences can be made by using site directed mutagenesis.

Nucleic acid sequences encoding the antibodies or binding members prepared by the method of the invention may be provided as constructs in the form of a plasmid, vector, transcription or expression cassette which comprises at least one nucleic acid as described above. The construct may be comprised within a recombinant host cell which comprises one or more constructs as above. Expression may conveniently be achieved by culturing, under appropriate conditions, recombinant host cells containing suitable nucleic acid sequences. Following expression, the antibody or antibody fragments may be isolated and/or purified using any suitable technique, then used as appropriate.

Systems for cloning and expression of a polypeptide in a variety of different host cells are well known. Suitable host cells include bacteria, mammalian cells, yeast, insect and baculovirus systems. Mammalian cell lines available in the art for expression of a heterologous polypeptide include Chinese hamster ovary (CHO) cells, HeLa cells, baby hamster kidney cells and NS0 mouse myeloma cells. A common, preferred bacterial host is *E. coli.* The expression of antibodies and antibody fragments in prokaryotic cells such as *E. coli* is well established in the art. Expression in eukaryotic cells in culture is also available to those skilled in the art as an option for production of a binding member.

General techniques for the production of antibodies are well known to the person skilled in the field, with such methods being discussed in, for example, Kohler and Milstein (1975) Nature 256: 495-497; US Patent No. 4,376,110; Harlow and Lane, Antibodies: a Laboratory Manual, (1988) Cold Spring Harbor. Techniques for the preparation of recombinant antibody molecules are described in the above references and also in, for example, European Patent Number 0,368,684.

In certain embodiments of the invention, recombinant nucleic acids comprising an insert coding for a heavy chain variable domain and/or for a light chain variable domain of antibodies or binding members are employed. By definition, such nucleic acids comprise encode single stranded nucleic acids, double stranded nucleic acids consisting of said coding nucleic acids and of complementary nucleic acids thereto, or these complementary (single stranded) nucleic acids themselves.

Furthermore, nucleic acids encoding a heavy chain variable domain and/or a light chain variable domain of antibodies can be enzymatically or chemically synthesised nucleic acids having the authentic sequence coding for a naturally-occurring heavy chain variable domain and/or for the light chain variable domain, or a mutant thereof.

An antibody prepared by the method of the invention may be produced by recombinant means, not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (i.e., cleaved by a signal peptidase) by the host cell. For prokaryotic host cells that do not recognize and process a native antibody signal sequence, the signal sequence is substituted by a prokaryotic signal sequence selected, for example, from the group of the alkaline phosphatase, penicillinase, Ipp, or heat-stable enterotoxin II leaders.

The term "isolated", when used in reference to the equinised antibodies prepared by the method of the invention, or to binding members derived therefrom, or polypeptides which encode the same, refers to the state in which said antibodies, binding members or nucleic acids (polynucleotides) are provided in an isolated and/or purified form, that is they have been separated, isolated or purified from their natural environment, and are provided in a substantially pure or homogeneous form, or, in the case of nucleic acid, free or substantially free of nucleic acid or genes of origin other than the sequence encoding a polypeptide with the required function. Accordingly, such isolated antibodies, binding members and isolated nucleic acids will be free or substantially free of material with which they are naturally associated, such as other polypeptides or nucleic acids with which they are found in their natural environment, or the environment in which they are prepared (e.g. cell culture) when such preparation is by recombinant DNA technology practised in vitro or in vivo.

Antibodies, binding members and nucleic acids may be formulated with diluents or adjuvants and still, for practical purposes, be considered as being provided in an isolated form. For example the antibodies and binding members can be mixed with gelatin or other carriers if used to coat microtitre plates for use in immunoassays, or will be mixed with pharmaceutically acceptable carriers or diluents when used in diagnosis or therapy. The antibodies or binding members may be glycosylated, either naturally or by systems of heterologous eukaryotic cells (e.g. CHO or NSO cells, or they may be (for example if produced by expression in a prokaryotic cell) unglycosylated (aglycosylated).

Heterogeneous preparations comprising anti-equine NGF equinised antibody molecules also form part of the disclosure. For example, such preparations may be mixtures of antibodies with full-length heavy chains and heavy chains lacking the C-terminal lysine, with various degrees of glycosylation and/or with derivatized amino acids, such as cyclization of an N-terminal glutamic acid to form a pyroglutamic acid residue.

### Pharmaceutical compositions

Typically the pharmaceutical compositions of the disclosure are formulated in a liquid formulation, a lyophilized formulation, a lyophilized formulation that is reconstituted as a liquid, or as an aerosol formulation. In certain instances, the antibody in the formulation is at a concentration of: about 0.5 mg/ml to about 250 mg/ml, about 0.5 mg/ml to about 45 mg/ml, about 0.5 mg/ml to about 100 mg/ml, about 100 mg/ml to about 200 mg/ml, or about 50 mg/ml to about 250 mg/ml.

In certain instances, the formulation further comprises a buffer. Typically the pH of the formulation is from about pH 5.5 to about pH 6.5. In certain instances, the buffer may comprise from about 4 mM to about 60 mM histidine buffer, about 5 mM to about 25 mM succinate buffer, or about 5 mM to 25 mM acetate buffer. In certain instances, the buffer comprises sodium chloride at a concentration of from about 10mM to 300mM, typically at around 125mM concentration and sodium citrate at a concentration of from about 5mM to 50mM, typically 25mM. In certain instances the formulation can further comprise a surfactant at a concentration of just above 0% to about 0.2%. In certain instances the surfactant is selected from the group consisting of, but not limited to: polysorbate-20, polysorbate-40, polysorbate-60, polysorbate-65, polysorbate-80, polysorbate-85, and combinations thereof. In a preferred instance, the surfactant is polysorbate-20 and may further comprise sodium chloride at a concentration of about 125mM and sodium citrate at a concentration of about 25mM.

### Administration

The antibodies or binding members of the present disclosure may be administered alone but will preferably be administered as a pharmaceutical composition which will generally comprise a suitable pharmaceutically acceptable excipient, diluent or carrier selected depending on the intended route of administration. Examples of suitable pharmaceutical carriers include; water, glycerol, ethanol and the like.

The monoclonal antibody or binding member of the present disclosure may be administered to an equine patient in need of treatment via any suitable route. Typically, the composition can be administered parenterally by injection or infusion. Examples of preferred routes for parenteral administration include, but are not limited to; intravenous, intracardial, intraarterial, intraperitoneal, intramuscular, intracavity, subcutaneous, transmucosal, inhalation or transdermal. Routes of administration may further include topical and enteral, for example, mucosal (including pulmonary), oral, nasal, rectal.

In instances where the composition is delivered as an injectable composition, for example in intravenous, intradermal or subcutaneous application, the active ingredient can be in the form of a parenterally acceptable aqueous solution which is pyrogen-free and has suitable pH, isotonicity and stability. Those of relevant skill in the art are well able to prepare suitable solutions using, for example, isotonic vehicles such as sodium chloride injection, Ringer's injection or, Lactated Ringer's injection. Preservatives, stabilisers, buffers, antioxidants and/or other additives may be included, as required.

The composition may also be administered via microspheres, liposomes, other microparticulate delivery systems or sustained release formulations placed in certain tissues including blood.

Examples of the techniques and protocols mentioned above and other techniques and protocols which may be used in accordance with the invention can be found in Remington's Pharmaceutical Sciences, 18th edition, Gennaro, A.R., Lippincott Williams & Wilkins; 20th edition ISBN 0-912734-04-3 and Pharmaceutical Dosage Forms and Drug Delivery Systems; Ansel, H.C. et al. 7th Edition ISBN 0-683305-72-7.

The antibodies and compositions of the disclosure are typically administered to a subject in a "therapeutically effective amount", this being an amount sufficient to show benefit to the subject to whom the composition is administered. The actual dose administered, and rate and time-course of administration, will depend on, and can be determined with due reference to, the nature and severity of the condition which is being treated, as well as factors such as the age, sex and weight of the subject being treated, as well as the route of administration. Further due consideration should be given to the properties of the composition, for example, its binding activity and in-vivo plasma life, the concentration of the antibody or binding member in the formulation, as well as the route, site and rate of delivery.

Dosage regimens can include a single administration of the antibody or composition of the disclosure, or multiple administrative doses of the antibody or composition. The antibody or antibody containing compositions can further be administered sequentially or separately with other therapeutics and medicaments which are used for the treatment of the condition for which the antibody or binding member of the present disclosure is being administered to treat.

Examples of dosage regimens which can be administered to a subject can be selected from the group comprising, but not limited to; 1µg/kg/day through to 20mg/kg/day, 1µg/kg/day through to 10mg/kg/day, 10µg/kg/day through to 1mg/kg/day. In certain instances, the dosage will be such that a plasma concentration of from 1µg/ml to 100µg/ml of the antibody is obtained. However, the actual dose of the composition administered, and rate and time-course of administration, will depend on the nature and severity of the condition being treated. Prescription of treatment, e.g. decisions on dosage etc, is ultimately within the responsibility and at the discretion of veterinary practitioners and other veterinary doctors, and typically takes account of the disorder to be treated, the condition of the individual patient, the site of delivery, the method of administration and other factors known to practitioners.

### Definitions

Unless otherwise defined, all technical and scientific terms used herein have the meaning commonly understood by a person who is skilled in the art in the field of the present invention. The meaning and scope of the terms should be clear, however, in the event of any ambiguity, definitions provided herein take precedent over any dictionary or extrinsic definition.

Throughout the specification, unless the context demands otherwise, the terms "comprise" or "include", or variations such as "comprises" or "comprising", "includes" or "including" will be understood to imply the inclusion of a stated integer or group of integers, but not the exclusion of any other integer or group of integers.

As used herein, terms such as "a", "an" and "the" include singular and plural referents unless the context clearly demands otherwise. Thus, for example, reference to "an active agent" or "a pharmacologically active agent" includes a single active agent as well as two or more different active agents in combination, while references to "a carrier" includes mixtures of two or more carriers as well as a single carrier, and the like. Further, unless otherwise required by context, singular terms shall include pluralities and plural terms shall include the singular.

As herein defined, the term "pain" means an unpleasant sensory and emotional experience associated with actual or potential tissue damage, or described in terms of such damage.

In relation to operative or post-operative pain, the US Animal Welfare Act (Animal Welfare Act 2002. AWA regulations, CFR, Title 9 (Animals and Animal Products), Chapter 1 (Animal and Plant Health Inspection Service, Department of Agriculture). Subchapter A (Animal Welfare), Parts 1-4) defines a painful procedure as any procedure that would reasonably be expected to cause more than slight or momentary pain or distress in a human being to which that procedure was applied, that is, pain in excess of that caused by injections or other minor procedures. Therefore, if an equine undergoes a painful surgical procedure, the animal should receive postoperative analgesics.

In further instance, an equine may be experiencing significant or chronic pain as a result of an associated medical condition such as an arthritic, for example polyarthritis, rheumatoid arthritis, inflammation, pruritis, osteoarthritis or a cancerous or malignant condition.

The term "nociception" refers to the perception of noxious stimuli. As herein defined "neuropathic pain" (also known as 'neuralgia') is a pain that comes from problems with signals from the nerves. It may arise as a consequence of a lesion or disease affecting the somatosensory system. There are causes of neuropathic pain and it may be associated with abnormal sensations called dysesthesia, which occur spontaneously. Alternatively, it may be associated with allodynia which results when the pain comes on, or gets worse, with a touch or stimulus that would not normally cause pain. For example, a slight touch on the face may trigger pain if you have trigeminal neuralgia, or the pressure of the bedclothes may trigger pain if you have diabetic neuropathy. Neuropathic pain may also result from allodynia, where the pain comes on, or gets worse, with a touch or stimulus that would not normally cause pain. For example, a slight touch to the face may trigger pain if a subject has trigeminal neuralgia. Neuropathic pain relating to hyperalgesia means that severe pain results from a stimulus or touch that would normally cause only slight discomfort, while paresthesia means that uncomfortable or painful feelings occur even when there is nothing in contact with the area causing the pain, for example pins and needles. Other forms of neuropathic pain involve pruritis or itch which can be associated with allergic or inflammatory responses in the skin and inflammatory pain resulting from tissue damage and repair processes.

As defined herein, the term "NGF neutralising antibody" or similar describes an antibody that is capable of neutralising the biological activation and signalling of NGF. The neutralising antibody, which may also be referred to as an antagonistic antibody, or a blocking antibody, specifically and preferably selectively, binds to NGF and inhibits one or more biological activities of NGF. For example, the neutralising antibody may inhibit the binding of a NGF to its target ligand, such as the cell membrane bound TrkA or p75 receptors.

As used herein, the term "biological activity" refers to any one or more inherent biological properties of a molecule (whether present naturally as found in vivo, or provided or enabled by recombinant means). Biological properties include but are not limited to receptor binding and/or activation; induction of cell signalling or cell proliferation, inhibiting cell growth, induction of cytokine production, induction of apoptosis, and enzymatic activity.

The term "complementarity determining region (CDR) ", as used herein, refers to amino acid sequences which together define the binding affinity and specificity of the natural Fv region of a native immunoglobulin binding site as delineated by Kabat et al. (Kabat,E.A., Wu,T.T., Perry,H., Gottesman,K. and Foeller,C. (1991) Sequences of Proteins of Immunological Interest, Fifth Edition. NIH Publication No. 91-3242). The term "framework region (FR)", as used herein, refers to amino acid sequences interposed between CDRs. These portions of the antibody serve to hold the CDRs in appropriate orientation (allows for CDRs to bind antigen).

The term "constant region (CR)" as used herein, refers to the portion of the antibody molecule which confers effector functions. In the present invention, constant regions typically mean equine constant regions, that is that the constant regions of the subject equinsed antibodies are derived from equine immunoglobulins. The heavy chain constant region can be selected from any equine heavy chain isotype.

The term "chimeric antibody" as used herein refers to an antibody containing sequences derived from two different antibodies, which typically are of different species. Most typically chimeric antibodies comprise variable domains derived from a donor specifies which bind specifically to a target epitope and constant domains derived from antibodies obtained from the target species to whom the antibody is to be administered.

The term "immunogenicity" as used herein refers to a measure of the ability of a targeting protein or therapeutic moiety to elicit an immune response (humoral or cellular) when administered to a recipient. The present invention is concerned with the immunogenicity of the subject equinised antibodies. Preferably the antibodies prepared by the method of the present invention have no immunogenicity, that is that no neutralising antibodies will be raised against them when administered to an equine, and further, no effector functions are mediated by the Fc regions of the antibody.

The term "identity" or "sequence identity" as used herein, means that at any particular amino acid residue position in an aligned sequence, the amino acid residue is identical between the aligned sequences. The term "similarity" or "sequence similarity" as used herein, indicates that, at any particular position in the aligned sequences, the amino acid residue is of a similar type between the sequences. For example, leucine may be substituted for an isoleucine or valine residue. This may be referred to as conservative substitution. Preferably when the amino acid sequences of the disclosure are modified by way of conservative substitution of any of the amino acid residues contained therein, these changes have no effect on the binding specificity or functional activity of the resulting antibody when compared to the unmodified antibody.

Sequence identity with respect to a (native) polypeptide of the disclosure and its functional derivative relates to the percentage of amino acid residues in the candidate sequence which are identical with the residues of the corresponding native polypeptide, after aligning the sequences and introducing gaps, if necessary, to achieve the maximum percentage homology, and not considering any conservative substitutions as part of the sequence identity. Neither N- or C-terminal extensions, nor insertions shall be construed as reducing sequence identity or homology. Methods and computer programs for performing an alignment of two or more amino acid sequences and determining their sequence identity or homology are well known to the person skilled in the art. For example, the percentage of identity or similarity of 2 amino acid sequences can be readily calculated using algorithms e.g. BLAST (Altschul et al. 1990), FASTA (Pearson & Lipman 1988), or the Smith-Waterman algorithm (Smith & Waterman 1981).

As used herein, reference to an amino acid residue having the "highest homology" to a second amino acid residue refers to the amino acid residue which has the most characteristics or properties in common with the second amino acid residue. In determining whether an amino acid residue has the highest homology to a second amino acid residue, an assessment may typically be made of factors such as, but not limited to, charge, polarity, hydrophobicity, side arm mass and side arm dimension.

The term "corresponding position" as used herein to refer to an amino acid residue that is present in a second sequence at a position corresponding to a specified amino acid residue in a first sequence is intended to refer to the position in the second sequence which is the same position as the position in the first sequence when the two sequences are aligned to allow for maximum sequence identity between the two sequences. Amino acid residues at corresponding positions have the same Kabat numbering.

The term "consists essentially of" or "consisting essentially of" as used herein means that a polypeptide may have additional features or elements beyond those described provided that such additional features or elements do not materially affect the ability of the antibody or antibody fragment to have binding specificity to equine NGF. That is, the antibody or antibody fragments comprising the polypeptides may have additional features or elements that do not interfere with the ability of the antibody or antibody fragments to bind to equine NGF and antagonise equine NGF functional activity. Such modifications may be introduced into the amino acid sequence in order to reduce the immunogenicity of the antibody. For example, a polypeptide consisting essentially of a specified sequence may contain one, two, three, four, five or more additional, deleted or substituted amino acids, at either end or at both ends of the sequence provided that these amino acids do not interfere with, inhibit, block or interrupt the role of the antibody or fragment in binding to equine NGF and sequestering its biological function. Similarly, a polypeptide molecule which contributes to the equine NGF antagonistic antibodies prepared by the method of the invention may be chemically modified with one or more functional groups provided that such functional groups do not interfere with the ability of the antibody or antibody fragment to bind to equine NGF and antagonise its function.

As used herein, the term "effective amount" or "therapeutically effective amount" means the amount of an agent, binding compound, small molecule, fusion protein or peptidomimetic of the disclosure which is required to suppress equine NGF binding to the p75 and/or TrkA receptors.

The terms "polypeptide", "peptide", or "protein" are used interchangeably herein to designate a linear series of amino acid residues connected one to the other by peptide bonds between the alpha-amino and carboxy groups of adjacent residues. The amino acid residues are usually in the natural "L" isomeric form. However, residues in the "D" isomeric form can be substituted for any L-amino acid residue, as long as the desired functional property is retained by the polypeptide.

As herein defined an "antibody" encompasses antigen-binding proteins which specifically bind to a target antigen of interest, in this case equine nerve growth factor, having one or more polypeptides that can be recombinantly prepared or which are genetically encodable by immunoglobulin genes, or fragments of immunoglobulin genes. The term "antibody" encompasses monoclonal and chimeric antibodies, in particular equinised antibodies, and further encompasses polyclonal antibodies or antibodies of any class or subtype. An "antibody" further extends to hybrid antibodies, bispecific antibodies, heteroantibodies and to functional fragments thereof which retain antigen binding.

The phrase "specifically binds to" refers to the binding of an antibody to a specific protein or target which is present amongst a heterogeneous population of proteins. Hence, when present in specific immunoassay conditions, the antibodies bind to a particular protein, in this case equine NGF, and do not bind in a significant amount to other proteins present in the sample.

As defined herein, an "equine" may also be referred to as a horse. Equines belong to the subspecies with the trinomial name Equus ferus caballus, these being hooved (ungulate) mammals. Equines are a subspecies of the family Equidae and include any species classified therein and extends to the over 300 breeds of horse known.

The present invention will now be described with reference to the following examples which are provided for the purpose of illustration and are not intended to be construed as being limiting on the present invention.

### EXAMPLES

### Example 1 - Production of antibodies

Whole antibody sequences were produced by combining equinised light chain and heavy chain variable domains of SEQ ID NO:1 and SEQ ID NO:2, respectively to C-terminal equine constant heavy chain or constant light chain domains. The equinised αD11 VH domain was combined with two different equine heavy chain constant domains; HC2 (IgG2) and HC6 (IgG6) and the equinised αD11 VL domain with the equine kappa light chain constant domain. The sequences of the full-length mature antibody chains are shown in SEQ ID 4 (light chain with kappa constant domain) and 6 (heavy chain with HC2 constant domain).

The combined amino acid sequences were converted to expressible form in mammalian cells by the optimal selection of codons and full chemical gene synthesis and cloning into a mammalian cell expression vector pcDNA3.1+.
The resultant cDNAs were transfected into CHO cells and the supernatants analysed as detailed in Examples 2 to 5.

### Example 2 - Determining binding of antibodies to murine and equine NGF

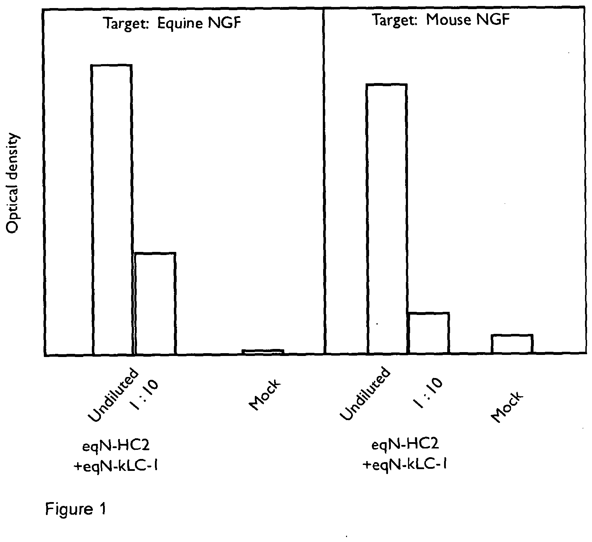

Equinised heavy and light chain cDNAs were transfected into CHO cells, the supernatants harvested and reacted in ELISA format with either equine or murine NGF. Following incubation and wash steps, the bound equine antibody was detected by reactivity with a goat-anti equine IgG specific polyclonal antibody linked to horseradish peroxidase (HRP) and developed using TMB. The optical density of the resulting product was measured at 450nm and compared with that from mock empty vector transfected supernatant (denoted as "Mock" in Figure 1).

The results are shown in the graph of Figure 1. Binding to mouse NGF is shown for the HC2 (IgG2 constant domain) equinised antibody (termed eqN-HC2+eqN-kLC-1). In the second part of the graph, binding of the eqN-HC2+eqN-kLC-1 antibody comprising the eqN-kLC-1 light chain and the eqN-HC2 (IgG2) constant chain to equine NGF is shown.

### Example 3 - Purification of equinised antibodies

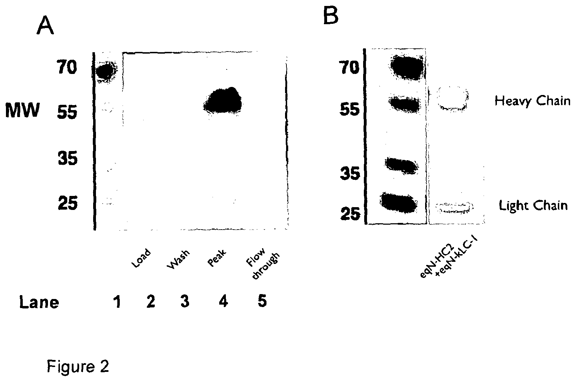

The supernatants obtained from Example 2 were purified using a Protein A column, separated by SDS-PAGE and tested for reactivity to anti-equine IgG polyclonal antibody HRP. The SDS-PAGE gel was also stained using Coomassie blue to detect heavy and light chains. The anti-equine IgG polyclonal antibody preparation predominantly recognises the equine heavy chains. The results are shown in Figure 2A and B.

The results show purification of equine anti-NGF with type 2 heavy chain by Protein A, as illustrated by a Western blot developed with anti-equine polyclonal antibody HRP. The peak fraction was analysed by Coomassie stained SDS-PAGE. Some degradation of the heavy and light chain is apparent by SDS-PAGE. The Coomassie blue stained gel (figure 2B, shows presence of heavy and light chains as well as complete antibody (MW of 70).

### Example 4 - Inhibition of NGF induced proliferation of TF-1 cells by equinised antibodies

Serial dilutions of CHO cell transfectant supernatants from Example 2 ("antagonist") were incubated with TF-1 cells in the presence of 1.0 ng/mL NGF. The resultant proliferation was measured by thymidine incorporation.

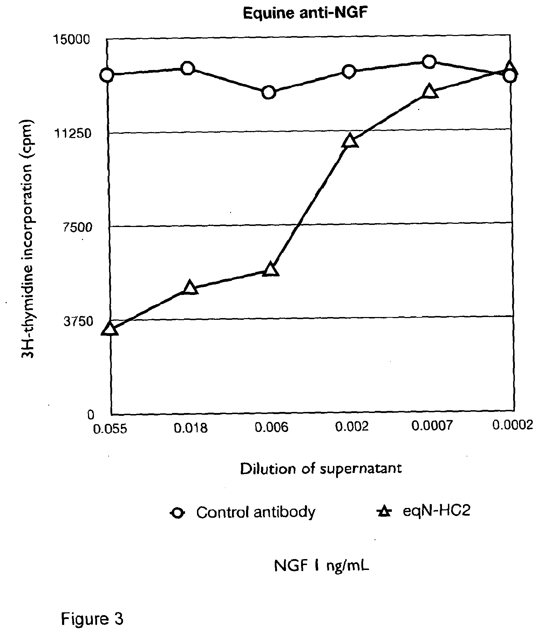

The results are shown in Figure 3. 50% inhibition was observed at a calculated 3-8 ng/mL monoclonal antibody (MAb) (antibody comprising the eqN-kLC-1 light chain and the eqN-HC2 (IgG2) constant chain).

### Example 5 - Complement deposition induced by antigen-captured equinised antibodies

Protein A purified transfectant supernatants from Example 2 were incubated with plates coated with 0.1 ng/mL NGF to capture the antibodies. The plates were washed and coated with 0.1 ng/mL NGF to capture the antibodies. The plates were washed and then incubated with human serum and bound complement C1q was measured by binding of anti-human C1q polyclonal antibody HRP and developed as above. The binding of C1q to antigen-captured "eqN-HC2 + eqN-kLC-1" was compared to a human anti-NGF MAb with human IgG1 Fc domain as positive control and an IgG4 variant as negative control.

### Complement Binding Method:

Plates were coated with 100 µl/well of 5 µg/ml mouse NGF and blocked with 5% BSA/PBS. Coated wells were incubated for 1 hour at room temperature with cell culture supernatants, containing recombinant equine anti-NGF IgG, diluted in PBS/1% BSA (100 µl/well). The plates were washed and incubated for 1 hour at room temperature with 100 µl/well of human serum diluted 1/100 in veronal buffered saline containing 0.5 mM MgCI2, 2 mM CaCI2, 0.05% Tween-20, 0.1% gelatin and 0.5% BSA. After washing, plates were incubated with 100 µl of a 1/800 dilution of sheep anti-C1q-HRP (Serotec) in PBS/1% BSA. After washing, plates were developed by the addition of 100µl TMB substrate (Thermo Scientific). Development was stopped by the addition of 100 µl of 2N H₂SO₄ and absorbance read at 450 nm.

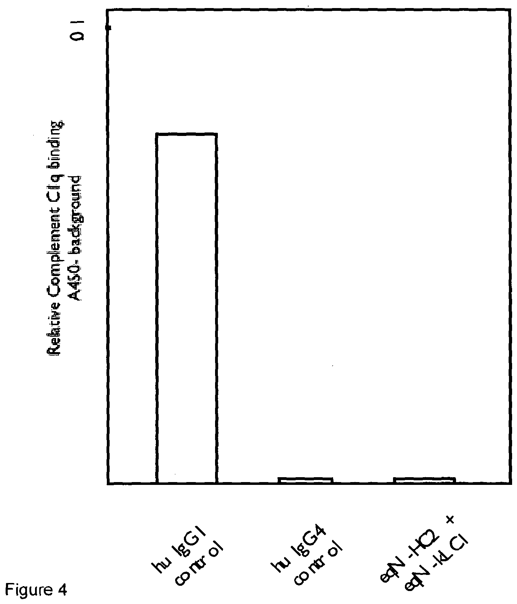

The results are shown in the graph of Figure 4. These results surprisingly show no binding of C1q to equinised HC2 antibodies (antibody comprising the eqN-kLC-1 light chain and the eqN-HC2 heavy chain (IgG2 constant domain heavy chain)). Hence, the results indicate that equinised antibodies with heavy chain constant domain type HC2 (Equine IgG2 derived constant domain) would be suitable for use in antagonising NGF, as NGF is a soluble mediator.

Accordingly, it is demonstrated herein, quite surprisingly, that where an antibody prepared by the method of the invention has an equine derived heavy chain of the HC2 (IgG2 equine heavy chain constant domain subtype), the binding of the antibody to equine NGF does not result in complement activation or other downstream effector functions, such as ADCC. Hence, said antibodies, in antagonising the biological functional activity of equine NGF by preventing binding of equine NGF to the membrane bound TrkA or p75 receptors, inhibit the associated downstream intracellular signalling cascade. Furthermore, as NGF expression frequently occurs in the proximity of nerves and the like, the NGF antagonising or neutralising antibodies prepared by the method of the invention, which have equine derived heavy chain of the HC2 (IgG2) subtype, can sequester equine NGF biological activity without recruiting a wider immune response. Such functional properties are unexpected, yet highly desirable.

### Example 6 - Preferential purification of equine IgG isotype HC2, but not HC6, using Protein A affinity chromatography

CHO cell supernatants resulting from transfection of the HC2 and HC6 variants of equinised αD11 were loaded onto a Protein A affinity column (as per Figure 2) and eluted fractions containing antibody were quantitated by binding to NGF by ELISA. As can be seen in Figure 10, the HC2 isotype, but not the HC6 isotype, was recovered using Protein A chromatography. These data suggest that Protein A chromatography can be a useful tool in the purification of HC2, but not HC6, isotypes of equine anti-NGF immunoglobulins.

### Example 7 - Anti-equine NGF monoclonal antibodies - safety and pyrexia

Anti-equine NGF monoclonal antibodies prepared by the method of this invention are expressed in CHO cells and purified by a combination of Protein A chromatography and/or size exclusion chromatography and are buffer exchanged into phosphate buffered saline. The antibodies are injected intravenously into horses at 0.01 - 10 mg/kg body weight and assessed for signs of toxicity by visual inspection by a veterinarian, change in body weight, body temperature and plasma biochemistry. No changes are expected to be observed in these or any plasma biochemistry analytes.

### Example 8 - Plasma pharmacokinetics of anti-equine NGF monoclonal antibodies in vivo - serum half-life and immunogenicity

The anti-equine NGF monoclonal antibodies prepared by the method of this invention are expressed in CHO cells and purified by a combination of Protein A chromatography and/or size exclusion chromatography and buffer exchanged into phosphate buffered saline. The antibodies are injected intravenously into horses in the range 0.01 - 10 mg/kg body weight and plasma samples are taken at various times over the next 2 weeks. Diluted plasma samples are assessed for anti-equine NGF antibody concentration by ELISA using NGF as the target and anti-equine polyclonal antibody-horseradish peroxidase secondary reagent. The plasma concentrations measured are consistent with two-phase kinetics, with a tissue distribution (alpha) phase and an elimination phase (beta) phase of several days.

The absence of a sharp decline in plasma concentration of anti-equine NGF antibody concentration between 100 and 300 hours is expected. This would demonstrate that there is neither pre-existing neutralising antibodies to recombinant anti-NGF monoclonal antibodies in horse blood nor are any such neutralising antibodies generated following infusion.

### Example 9 - Anti-equine NGF monoclonal antibodies reduce inflammatory pain due to osteoarthritis in vivo

Groups of osteoarthritic horses are injected intravenously or intra-articularly with either anti-equine NGF monoclonal antibodies of this patent at 0.01 - 10 mg/kg body weight or phosphate buffered saline as vehicle control (= day 0). The horses are assessed for lameness over 4- 14 days by a visual scoring method (e.g. score 0, no lameness (full weight bearing); score 1, slight lameness (not full weight bearing but walking well); score 2, moderate lameness (slightly weight bearing and not walking well), score 3, severe lameness (not weight bearing)). Observers are blinded to which horses receive which injection.

Lameness scores are expected to be reduced in the horses receiving anti-equine NGF monoclonal antibodies over time post-injection compared with vehicle control, indicating that the anti-equine NGF monoclonal antibodies will have an effect in reducing the pain in the horses over that seen with vehicle alone.

### Example 10 - Comparison example showing the effect of anti-canine NGF monoclonal antibodies in reducing inflammatory pain in vivo

### Antibody therapy:

The method of preparing antibodies of the present invention was applied to produce a caninised antibody suitable for use in canines. A caninised αD11 VL domain was combined with a canine kappa light chain constant domain and a caninised αD11 VH domain was combined with a canine heavy chain isotype. Anti-canine NGF monoclonal antibodies derived from expression vectors expressing the heavy and light chains were expressed in CHO cells and purified by a combination of ion exchange chromatography, hydrophobic interaction chromatography and size exclusion chromatography and buffer exchanged into phosphate buffered saline.

### Canine model of inflammation:

All experiments were carried out with prior approval of the Institutional Ethics Committee (CRL, Ireland). Beagle dogs were injected (= day -1) with kaolin into the footpad of one hind leg in order to generate a self-resolving inflammation beginning approximately 24 hours later and which causes the dogs to become temporarily lame. In this model, once the initial inflammation response to kaolin recedes, the dogs become steadily less lame over the period of approximately 1-2 weeks and then make a full recovery.

Groups of 3 dogs were injected intravenously with either anti-canine NGF monoclonal antibodies at 200 µg/kg body weight or phosphate buffered saline as vehicle control (= day 0). The dogs were assessed for lameness over 7 days by a visual scoring method (score 0, no lameness (full weight bearing); score 1, slight lameness (not full weight bearing but walking well); score 2, moderate lameness (slightly weight bearing and not walking well), score 3, severe lameness (not weight bearing)). Observers were blinded to which dogs received which injection.

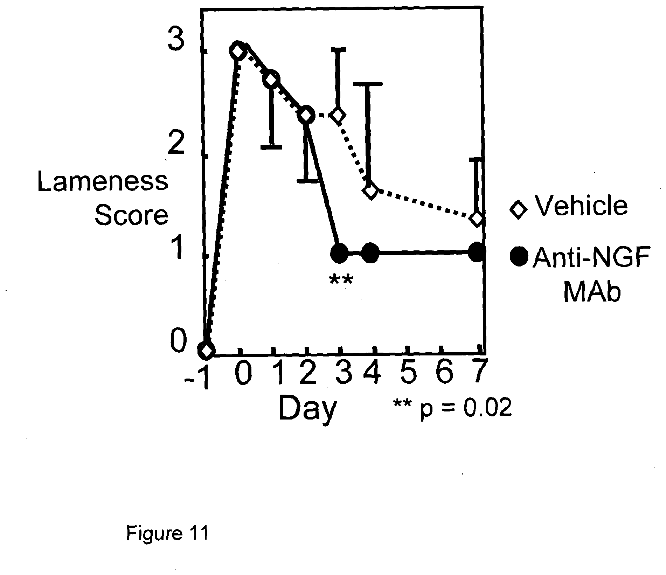

The results are shown in Figure 11. Lameness scores were reduced in the dogs receiving anti-NGF monoclonal antibodies by day 3 post-injection compared with vehicle control, indicating that the anti-NGF monoclonal antibodies had an effect in reducing the pain in the dogs over that seen with vehicle alone. The delayed activity is consistent with the plasma pharmacokinetics of anti-canine NGF monoclonal antibodies which demonstrated a slow tissue distribution (alpha) phase of approximately 30 hours and the relatively poor vascularisation of the footpad area. The results shown in Figure 11 show that the anti-canine NGF antibodies prepared by a method corresponding to the method of the present invention reduce inflammatory pain in dogs with a consequent reduction in lameness.

## Claims

1. A method of preparing an antibody suitable for use in an equine comprising the steps of:
- providing a donor antibody from a species other than an equine, wherein the donor antibody has binding specificity for a target antigen present in equines;
- comparing each amino acid residue of the amino acid sequence of framework regions of the donor antibody with each amino acid residue present at a corresponding position in the amino acid sequence of framework regions of a plurality of equine antibodies to identify one or more amino acid residues within the amino acid sequence of the framework regions of the donor antibody that differ from one or more amino acid residues at the corresponding position within the amino acid sequence of framework regions of the plurality of equine antibodies; and
- substituting the one or more identified amino acid residues in the donor antibody with the one or more amino acid residues present at the corresponding position in the plurality of equine antibodies to produce a modified antibody,
wherein the modified antibody does not contain any amino acid in any position within the framework regions which would be foreign at the corresponding position in equines,
wherein the modified antibody is capable of specifically binding to equine nerve growth factor (NGF) and inhibiting the ability of equine NGF to bind to the p75 or TrkA equine NGF receptor; and
wherein substitution of an amino acid residue in the donor antibody is undertaken using the principle of conservative substitution.

## Patentansprüche

1. Verfahren zur Herstellung eines Antikörpers, der zur Verwendung in einem equinen geeignet ist, umfassend die Schritte:
- Bereitstellen eines Donorantikörpers aus einer anderen Spezies als einem equinen, wobei der Donorantikörper Bindungsspezifität für ein Targetantigen hat, das in equinen vorliegt;
- Vergleichen jedes Aminosäurerestes der Aminosäuresequenz von Gerüstregionen des Donorantikörpers mit jedem Aminosäurerest, der an einer entsprechenden Position in der Aminosäuresequenz von Gerüstregionen einer Vielzahl von equinen Antikörpern vorliegt, um einen oder mehrere Aminosäurerest(e) innerhalb der Aminosäuresequenz der Gerüstregionen des Donorantikörpers zu identifizieren, der/die sich von einem oder mehreren Aminosäurerest(en) an der entsprechenden Position in der Aminosäuresequenz von Gerüstregionen der Vielzahl equiner Antikörper unterscheidet/unterscheiden, und
- Substituieren des einen identifizierten Aminosäurerestes oder der mehreren identifizierten Aminosäurereste in dem Donorantikörper durch den einen Aminosäurerest oder die mehreren Aminosäurereste, der/die an der entsprechenden Position in der Vielzahl equiner Antikörper vorliegt/vorliegen, um einen modifizierten Antikörper zu produzieren,
wobei der modifizierte Antikörper keine Aminosäure in einer beliebigen Position innerhalb der Gerüstregionen enthält, die an der entsprechenden Position in equinen fremd wäre,
wobei der modifizierte Antikörper fähig ist, spezifisch an equinen-Nervenwachstumsfaktor (NGF) zu binden und die Fähigkeit von equinen-NGF, an den p75- oder TrkA-equinen-NGF-Rezeptor zu binden, zu inhibieren, und
wobei eine Substitution eines Aminosäurerestes in dem Donorantikörper unter Verwendung des Prinzips der konservativen Substitution durchgeführt wird.

## Revendications

1. Procédé de préparation d'un anticorps approprié pour une utilisation chez un équin comprenant les étapes suivantes :
- la fourniture d'un anticorps donneur provenant d'une espèce autre qu'un équin, dans lequel l'anticorps donneur a une spécificité de liaison à un antigène cible présent chez les équins ;
- la comparaison de chaque résidu d'acide aminé de la séquence d'acides aminés des régions charpentes de l'anticorps donneur avec chaque résidu d'acide aminé présent sur une position correspondante dans la séquence d'acides aminés de régions charpentes d'une pluralité d'anticorps équins pour identifier un ou plusieurs résidus d'acides aminés dans la séquence d'acides aminés des régions charpentes de l'anticorps donneur qui diffèrent des un ou plusieurs résidus d'acides aminés sur une position correspondante dans la séquence d'acides aminés de régions charpente de la pluralité d'anticorps équins ; et
- la substitution des un ou plusieurs résidus d'acides aminés identifiés dans l'anticorps donneur avec les un ou plusieurs résidus d'acides aminés présents sur la position correspondante dans la pluralité d'anticorps équins pour produire un anticorps modifié,
dans lequel l'anticorps modifié ne contient pas d'acide aminé dans une position quelconque dans les régions charpentes qui serait étranger sur la position correspondante chez les équins,
dans lequel l'anticorps modifié est capable de se lier spécifiquement au facteur de croissance des nerfs (NGF) d'équin et d'inhiber la capacité du NGF d'équin à se lier au récepteur de NGF d'équin p75 ou TrkA ; et
dans lequel une substitution d'un résidu d'acide aminé dans l'anticorps donneur est entreprise en appliquant le principe de substitution conservatrice.
